(19) Europäisches Patentamt
European Patent Office
Office européen des brevets

(11) EP 3 644 317 B1

(12) **EUROPEAN PATENT SPECIFICATION**

(45) Date of publication and mention of the grant of the patent:
**21.12.2022 Bulletin 2022/51**

(21) Application number: **19197488.0**

(22) Date of filing: **16.09.2019**

(51) International Patent Classification (IPC):
***G16B 15/10** (2019.01)*

(52) Cooperative Patent Classification (CPC):
**G16B 15/10;** G16B 50/30

(54) **DEVICE, METHOD, AND PROGRAM FOR SEARCHING COMPOUND**

VORRICHTUNG, VERFAHREN UND PROGRAMM ZUM SUCHEN EINER VERBINDUNG

DISPOSITIF, PROCÉDÉ ET PROGRAMME DE RECHERCHE DE COMPOSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 JP 2018201591**

(43) Date of publication of application:
**29.04.2020 Bulletin 2020/18**

(73) Proprietor: **FUJITSU LIMITED**
**Kanagawa 211-8588 (JP)**

(72) Inventors:
- **SHIBASAKI, Takayuki**
  **Kawasaki-shi, Kanagawa 211-8588 (JP)**
- **UEMURA, Taiki**
  **Kawasaki-shi, Kanagawa 211-8588 (JP)**

(74) Representative: **Haseltine Lake Kempner LLP**
**Cheapside House**
**138 Cheapside**
**London EC2V 6BJ (GB)**

(56) References cited:
- **Ryan Babbush ET AL: "Construction of Energy Functions for Lattice Heteropolymer Models: A Case Study in Constraint Satisfaction Programming and Adiabatic Quantum Optimization Contents", , 13 June 2013 (2013-06-13), XP055676554, Retrieved from the Internet: URL:https://arxiv.org/pdf/1211.3422.pdf [retrieved on 2020-03-12]**
- **ALEJANDRO PERDOMO-ORTIZ ET AL: "Finding low-energy conformations of lattice protein models by quantum annealing", SCIENTIFIC REPORTS, vol. 2, no. 1, 13 August 2012 (2012-08-13) , XP055676849, DOI: 10.1038/srep00571**
- **ALEJANDRO PERDOMO ET AL: "On the construction of model Hamiltonians for adiabatic quantum computation and its application to finding low energy conformations of lattice protein models", ARXIV.ORG, CORNELL UNIVERSITY LIBRARY, 201 OLIN LIBRARY CORNELL UNIVERSITY ITHACA, NY 14853, 23 January 2008 (2008-01-23), XP080349362, DOI: 10.1103/PHYSREVA.78.012320**
- **ALESSANDRO DAL PALÙ ET AL: "A constraint solver for discrete lattices, its parallelization, and application to protein structure prediction", SOFTWARE PRACTICE & EXPERIENCE, WILEY & SONS, BOGNOR REGIS, GB, vol. 37, no. 13, 10 November 2007 (2007-11-10), pages 1405-1449, XP007917643, ISSN: 0038-0644, DOI: 10.1002/SPE.810 [retrieved on 2007-03-12]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

FIELD

The embodiments discussed herein relate to a method, device, and program for searching a compound.

BACKGROUND

A protein is a chain polymer where amino acids are linked one-dimensionally without branching. A protein forms a certain conformation (three-dimensional shape) by folding a chain polymer thereof. A conformation of a protein is determined by a sequence of amino acids.

A conformation of a protein is deeply related to functions of a protein. A molecule-recognition function of a protein is expressed by specifically bounding a certain region within a conformation thereof to a certain molecule. Therefore, it is important to determine a conformation of a protein to understand functions of the protein.

For example, a conformation of a protein can be determined by X-ray crystallography, or nuclear magnetic resonance spectroscopy (NMR). However, it takes a long time to determine a conformation of one protein by X-ray crystallography or NMR. According to X-ray crystallography, moreover, a single crystal of one kind of protein is created first. When the single crystal cannot be created, X-ray crystallography cannot be performed on a conformation of the protein. Moreover, NMR can determine a conformation of a protein in an aqueous solution without crystalizing the protein, but a large quantity of information related to a conformation of the protein cannot be obtained when the protein is a large protein.

Meanwhile, a sequence of amino acids of a protein can be relatively easily determined from genetic information or the protein itself, even when a conformation of the protein is unknown.

Accordingly, there have been attempts to predict a conformation of a protein from a sequence of amino acids. For example, there is a method for determining folding of a protein according to the diamond encoding method. The method is a method for embedding positions of chain amino acids in a diamond lattice, and can express a three-dimensional structure (conformation). Energy of the conformation determined by the above-described method can be calculated, for example, using the Ising model. To solve the Ising model, for example, an annealing machine is used. One example of the background technology is disclosed in R. Babbush et.al., Construction of Energy Functions for Lattice Heteropolymer Models: A Case Study in Constraint Satisfaction Programming and Adiabatic Quantum Optimization, arXiv:quant-ph/1211.3422v2 (https://arxiv.org/abs/1211.3422).

SUMMARY

Since there is a restriction in hardware of an anealing machine for solving an Ising model, there are restrictions in the number of arithmetic bits or quantum bits the annealing machine can handle.

Meanwhile, the number of bits used for solving a problem of folding of a protein increases exponentially relative to a scale of the protein (the number of amino acid residues) as demonstrated in the graph of FIG. 1.

As described above, a scale of a problem to be solved is limited by the restriction in the number of bits handled by hardware, and therefore search targets of amino acids cannot be expanded.

The invention is defined in the independent claims, to which reference should now be made. Advantageous developments are set out in the dependent claims.

According to one aspect of the present disclosure, provided is a device for searching a compound, which can appropriately suppress the number of arithmetic bits or quantum bits used for searching a predetermined compound, and can search a compound having a large molecular weight.

According to another aspect of the present disclosure, provided is a method for searching a compound, which can appropriately suppress the number of arithmetic bits or quantum bits used for searching a predetermined compound, and can search a compound having a large molecular weight.

According to another aspect of the present disclosure, provided is a program for searching a compound, which can appropriately suppress the number of arithmetic bits or quantum bits used for searching a predetermined compound, and can search a compound having a large molecular weight.

BRIEF DESCRIPTION OF DRAWINGS

The invention is described, by way of example only, with reference to the following drawings, in which:

FIG. 1 is a graph depicting a relationship between the number of amino acid residues and the number of bits used.
FIG. 2A is a schematic view for searching a stable conformation of a protein (part 1).

FIG. 2B is a schematic view for searching the stable conformation of the protein (part 2).
FIG. 2C is a schematic view for searching the stable conformation of the protein (part 3).
FIG. 3A is a schematic view for describing the diamond encoding method (part 1).
FIG. 3B is a schematic view for describing the diamond encoding method (part 2).
FIG. 3C is a schematic view for describing the diamond encoding method (part 3).
FIG. 3D is a schematic view for describing the diamond encoding method (part 4).
FIG. 3E is a schematic view for describing the diamond encoding method (part 5).
FIG. 4 is a conceptual view illustrating a state where a lattice space is limited according to the disclosed technology.
FIG. 5 is a view illustrating an example where amino acid residues are arranged on the outermost edge (outer shell) of the limited lattice space.
FIG. 6 is a view illustrating a structural example of the disclosed device for searching a compound.
FIG. 7 is a flowchart for describing a method for searching a stable conformation of a protein using the device for searching a compound 10A of FIG. 6.
FIG. 8 is a view illustrating a case where each lattice within a radius r is $S_r$.
FIG. 9A is a view illustrating a collection of lattice points to which amino acid residues move in the case where a limited lattice space is not generated (part 1).
FIG. 9B is a view illustrating a collection of lattice points to which amino acid residues move in the case where a limited lattice space is not generated (part 2).
FIG. 9C is a view illustrating a collection of lattice points to which amino acid residues move in the case where a limited lattice space is not generated (part 3).
FIG. 9D is a view illustrating a collection of lattice points to which amino acid residues move in the case where a limited lattice space is not generated (part 4).
FIG. 10 is a view illustrating $S_1$, $S_2$, and $S_3$ three-dimensionally.
FIG. 11A is a view illustrating one example of a state where space information is assigned to each of bits $X_1$ to $X_n$ (part 1).
FIG. 11B is a view illustrating one example of the state where space information is assigned to each of bits $X_1$ to $X_n$ (part 2).
FIG. 11C is a view illustrating one example of the state where space information is assigned to each of bits $X_1$ to $X_n$ (part 3).
FIG. 12 is a view for describing $H_{one}$.
FIG. 13 is a view for describing $H_{conn}$.
FIG. 14 is a view for describing $H_{olap}$.
FIG. 15A is a view for describing $H_{pair}$ (part 1).
FIG. 15B is a view for describing $H_{pair}$ (part 2).
FIG. 16 is a view illustrating one example of a weight file.
FIG. 17 is a view illustrating a conceptual structure of an optimizing device (arithmetic unit) used for simulated annealing.
FIG. 18 is a block diagram of a circuit level of a transition controlling unit.
FIG. 19 is a diagram illustrating an operation flow of a transition controlling unit.
FIG. 20A is a view illustrating one example of an arrangement of amino acid residues in a diamond lattice space (Example 1).
FIG. 20B is a view illustrating one example where the diamond lattice space of FIG. 20A is expanded (Example 1).
FIG. 21 is a view illustrating another structural example of the disclosed device for searching a compound (Example 2).
FIG. 22 is a flowchart illustrating another method for searching a stable structure of a protein using the device for searching a compound 10B of FIG. 21 (Example 2).
FIG. 23A is a view illustrating one example of an arrangement of amino acid residues in a diamond lattice space (Example 2).
FIG. 23B is a view illustrating one example where the diamond lattice space of FIG. 23A is expanded (Example 2).
FIG. 23C is a view illustrating a state where Nos. 1 to 4 amino acid residues are fixed in the expanded diamond lattice space of FIG. 23B (Example 2).
FIG. 24A is a view illustrating one example of an arrangement of amino acid residues in a diamond lattice space (Example 3).
FIG. 24B is a view illustrating one example where the diamond lattice space of FIG. 24A is expanded (Example 3).
FIG. 24C is a view illustrating a state where Nos. 1 to 4 amino acid residues are fixed in the expanded diamond lattice space of FIG. 24B (Example 3).
FIG. 25A is a view illustrating one example of an arrangement of amino acid residues in a diamond lattice space (Example 4).
FIG. 25B is a view illustrating one example where the diamond lattice space of FIG. 25A is expanded (Example 4).

FIG. 26A is a view illustrating one example of an arrangement of amino acid residues in a diamond lattice space (Example 5).
FIG. 26B is a view illustrating one example where the diamond lattice space of FIG. 26A is expanded (Example 5).
FIG. 26C is a view illustrating a state where Nos. 1 to 4 amino acid residues are fixed in the expanded diamond lattice space of FIG. 26B (Example 5).
FIG. 27A is a view illustrating one example of an arrangement of amino acid residues in a diamond lattice space (Example 6).
FIG. 27B is a view illustrating one example where the diamond lattice space of FIG. 27A is expanded (Example 6).
FIG. 27C is a view illustrating a state where Nos. 1 to 4 amino acid residues are fixed in the expanded diamond lattice space of FIG. 27B (Example 6).
FIG. 28 is a diagram summarizing relationships of Examples 1 to 6.
FIG. 29 is a view illustrating another structural example of the disclosed device for searching a compound.
FIG. 30 is a flowchart of a modified example of the steps S101 to S107 of the flowchart of FIG. 7.
FIG. 31 is a view for describing the limit in an alignment of amino acid residues when a straight chain number limiting parameter M (part 1) is set.
FIG. 32 is a view for describing the limit in an alignment of amino acid residues when a straight chain number limiting parameter M (part 2) is set.
FIG. 33 is a flowchart of a modified example of the steps S101 to S107 of the flowchart of FIG. 7.
FIG. 34 is a view for describing the maximum space when a straight chain number limiting parameter M.
FIG. 35 is a graph comparing the number of bits used between Comparative Example and Referential Examples.
FIG. 36 is a view describing one example of an effect of reducing the number of bits.

DESCRIPTION OF EMBODIMENTS

The disclosed device for searching a compound is a compound search device for searching a compound in which a plurality of compound groups are linked with one another.

The device for searching a compound includes at least a defining unit, a limiting unit, an assigning unit, an arithmetic unit, a judging unit, and a controlling unit.

The defining unit is configured to define a lattice space that is a collection of lattices where a plurality of compound groups are sequentially arranged.

The limiting unit is configured to, in the case where any of the compound groups is arranged in any of the lattices of the lattice space, followed by arranging a next compound group in the lattice space, generate a limited lattice space that is a space created by eliminating, from the lattice space, undesirable regions for the next compound group to be arranged.

The assigning unit is configured to assign a bit to each of lattice points, to which the compound groups can be arranged, in the limited lattice space.

The arithmetic unit is configured to perform a ground state search on an Ising model obtained through conversion based on restriction conditions related to each of the lattice points according to simulated annealing, to thereby calculate minimum energy of the Ising model.

The judging unit is configured to judge whether any of the compound groups assigned to the lattice points is arranged on the outermost edge of the limited lattice space or not.

The controlling unit is configured to cause the limiting unit to execute expansion of the limited lattice space, causing the assigning unit to execute assignment of the bits to the lattice points included in the limited lattice space after the expansion, and cause the arithmetic unit to execute calculation of the minimum energy of the Ising model, in the case where the judging unit judges that any of the compound groups assigned to the lattice points is arranged on the outermost edge of the limited lattice space.

The disclosed method for searching a compound is a method for searching a compound in which a plurality of compound groups are linked with one another.

The method for searching a compound allows a computer to perform a method including: defining a lattice space that is a collection of lattices where a plurality of compound groups are sequentially arranged; in the case where any of the compound groups is arranged in any of the lattices of the lattice space, followed by arranging a next compound group in the lattice space, generating a limited lattice space that is a space created by eliminating, from the lattice space, undesirable regions for the next compound group to be arranged; assigning a bit to each of lattice points, to which the compound groups can be arranged, in the limited lattice space; and performing a ground state search on an Ising model obtained through conversion based on restriction conditions related to each of the lattice points according to simulated annealing, to thereby calculate minimum energy of the Ising model.

In the method for searching method, moreover, the computer judges whether any of the compound groups assigned to the lattice points is arranged on the outermost edge of the limited lattice space or not, and executes expansion of the limited lattice space, assigning the bits to the lattice points included in the limited lattice space after the expansion, assignment of the bits to the lattice points included in the limited lattice space after the expansion, and calculation of the minimum energy of the Ising model, in the case where it is judged that any of the compound groups assigned to the lattice points is arranged on the outermost edge of the limited lattice space.

The disclosed program for searching a compound is a program for causing a computer to execute a method for searching a compound in which a plurality of compound groups are linked with one another.

The method includes: defining a lattice space that is a collection of lattices where a plurality of compound groups are sequentially arranged; in the case where any of the compound groups is arranged in any of the lattices of the lattice space, followed by arranging a next compound group in the lattice space, generating a limited lattice space that is a space created by eliminating, from the lattice space, undesirable regions for the next compound group to be arranged; assigning a bit to each of lattice points, to which the compound groups can be arranged, in the limited lattice space; and performing a ground state search on an Ising model obtained through conversion based on restriction conditions related to each of the lattice points according to simulated annealing, to thereby calculate minimum energy of the Ising model.

In the program for searching method, moreover, the computer judges whether any of the compound groups assigned to the lattice points is arranged on the outermost edge of the limited lattice space or not, and executes expansion of the limited lattice space, assigning the bits to the lattice points included in the limited lattice space after the expansion, assignment of the bits to the lattice points included in the limited lattice space after the expansion, and calculation of the minimum energy of the Ising model, in the case where it is judged that any of the compound groups assigned to the lattice points is arranged on the outermost edge of the limited lattice space.

Before describing the details of the disclosed technology, a method for determining folding of a protein that is a compound according to the diamond encoding method will be described.

A search of a stable conformation of a protein is typically performed in the following manner.

First, coarse graining of a protein is performed (FIG. 2A). For example, the coarse graining of a protein is performed by coarse graining atoms 2 constituting the proteins into amino acid residue units 1A, 1B, and 1C.

Next, a structure search is performed using the created coarse-grained model (FIG. 2B). The structure search is performed according to the diamond encoding method described later.

Next, the coarse-grained model is returned back to the whole atoms (FIG. 2C).

The diamond encoding method is a method where a linear amino acid is embedded in a position on a diamond lattice, and can represents a three-dimensional structure. For the sake of simplicity, a two-dimensional structure is described as an example.

Used as an example is a linear pentapeptide having a structure illustrated in FIG. 3A, where 5 amino acid residues are linked, when the structure is represented by a linear structure. In FIGs. 3A to 3E, a number in each circle is a number of the amino acid residue in the linear pentapeptide.

First, an amino acid residue of No. 1 is arranged at a center of a diamond lattice as illustrated in FIG. 3A, positions where an amino acid residue of No. 2 can be arranged are limited to positions next to the center as illustrated in FIG. 3B (the positions numbered as 2).

Next, in FIG. 3C, positions to which an amino acid residue of No. 3 bonded to next to the amino acid residues of No. 2 can be arranged are limited to positions next to the positions numbered as 2 (the positions numbered as 3) in FIG. 3B.

Next, in FIG. 3D, positions to which an amino acid residue of No. 4 bonded to next to the amino acid residues of No. 3 can be arranged are limited to positions next to the positions numbered as 3 (the positions numbered as 4) in FIG. 3C.

Next, in FIG. 3E, positions to which an amino acid residue of No. 5 bonded to next to the amino acid residues of No. 4 can be arranged are limited to positions next to the positions numbered as 4 (the positions numbered as 5) in FIG. 3D.

In the manner as described above, a three-dimensional structure can be expressed by linking the positions where amino acid residues can be arranged.

As amino acid residues are bounded into a straight chain, a radius (n) of a diamond lattice space is set according to the number (n) of amino acid residues to be bounded.

However, amino acid residues are typically rarely arranged into a straight chain in a protein due to interaction between amino acid residues.

Therefore, a conformation of a protein can be determined without matching a radius r of a diamond lattice space with the number (n) of amino acid residues as illustrated in FIG. 4.

According to the disclosed technology, therefore, in the case where any of the compound groups is arranged in any of the lattices of the lattice space, followed by arranging a next compound group in the lattice space, a limited lattice space that is a space created by eliminating, from the lattice space, undesirable regions for the next compound group to be arranged, is generated, and a bit is assigned to each of lattice points, to which the compound groups can be arranged in the limited lattice space. This technology may be referred to as "Referential Example" hereinafter. As a result, the number of arithmetic bits or quantum bits used for a search of a predetermined compound is suppressed, and a compound having a large molecular weight can be searched.

In this case, however, the arrangement of the compound group is limited by the outermost edge of the limited lattice space if the limited lattice space is too small. As a result, an appropriate conformation may not be obtained. Specifically, in the case where any of the compound groups assigned to the lattice points is arranged on the outermost edge of the limited lattice space, the lattice space may be excessively limited.

For example, an example where 5 amino acid residues are aligned in a diamond lattice space having a radius of 3 as illustrated in FIG. 5 is considered. Since the third amino acid residue is arranged on the outermost edge, the fourth amino acid residues is not arranged in the directions indicated with the arrows. However, the more stable conformation may be obtained when the fourth amino acid residue is arranged in any of the directions of the arrows.

In the disclosed technology, therefore, the limited lattice space is expanded further when any of the compound groups assigned to the lattice groups is arranged on the outermost edge of the limited lattice space. As a result, the number of arithmetic bits or quantum bits used for search of a predetermined compound can be appropriately suppressed, and a compound having a large molecular weight can be searched.

In the present specification, the term "outermost edge" means an outer shell of a diamond lattice space, and includes both the outermost surface and the outermost side.

For example, the compound groups are amino acid residues.

In the case where the compound groups are amino acid residues, examples of the compound include a protein.

Amino acid that is a base of an amino acid residue may be natural amino acid or synthetic amino acid. Examples of the natural amino acid include alanine, arginine, asparagine, aspartic acid, cysteine, glutamine, glutamic acid, glycine, histidine, isoleucine, leucine, lysine, methionine, phenyl alanine, proline, serine, threonine, tryptophan, tyrosine, valine, β-alanine, and β-phenylalanine. Examples of the synthetic amino acid include para-benzoylphenylalanine.

The number of amino acid residues in the protein is not particularly limited and may be appropriately selected depending on the intended purpose. For example, the number thereof may be from about 10 to about 30, or about several hundreds.

For example, the number thereof may be from about 10 to about 30 as long as the protein is a protein for middle molecule drug discovery.

One example of the disclosed technology will be described using examples of a device, flowcharts, etc., hereinafter.

A structural example of a device for searching a compound is illustrated in FIG. 6.

The device for searching a compound 10A illustrated in FIG. 6 includes a compound group number counting unit 11, a defining unit 12, a limiting unit 13, an assigning unit 14, a H generating unit 15, a weight extracting unit 16, a weight file creating unit 17, an arithmetic unit 18, a judging unit 19, a controlling unit 20, and an outputting unit 21.

A flowchart for describing a method for searching a stable conformation of a protein using the device for searching a compound 10A of FIG. 6 is illustrated in FIG. 7.

<Step S101>

First, the number (n) of amino acid residues (compound groups) constituting the input protein (an alignment of the amino acid residues) is counted by the compound group number counting unit 11 (S101).

<Step S102>

Next, a lattice space that is a collection of lattices to which a plurality of the amino acid residues are sequentially arranged is defined by the defining unit 12 based on the number (n) of the amino acid residues (S102).

One example of the definition of the lattice space will be described. The lattice space is three dimensional, but a two dimensional lattice space is described as an example for simplicity.

First, a collection of lattices within a radius r in a diamond lattice space is determined as a shell, and each lattice point is determined as $S_r$. Each lattice point $S_r$ is represented as in FIG. 8.

In the case where a limited lattice space is not generated unlike the disclosed technology, for example, collections $V_1$ to $V_5$ of lattice points to which amino acid residues of Nos. 1 to 5 are moved is represented as in FIGs. 9A to 9D.

In FIG. 9A, $V_1 = S_1$, and $V_2 = S_2$.

In FIG. 9B, $V_3 = S_3$.

In FIG. 9C, $V_4 = S_2, S_4$.

In FIG. 9D, $V_5 = S_3, S_5$.

Note that, when $S_1$, $S_2$, and $S_3$ are represented in three dimension, $S_1$, $S_2$, and $S_3$ are represented as in FIG. 10. In FIG. 10, $A = S_1$, $B = S_2$, and $C = S_3$.

In the case where a limited lattice space is not generated, a space $V_i$ used for i-numbered amino acid residues in a protein having amino acid residues in the number of n is represented by the following formula.

$$V_i = \bigcup_{r \in J} S_r$$

In the formula above, i = {1, 2, 3, ......n}.

In case of an odd numbered (i = odd number) amino acid residue, J = {1, 3, .....i}. In case of an even numbered (i = even number) amino acid residue, J = {2, 4, .....i}.

<Steps S103 and S104>

In the disclosed technology, meanwhile, in the case where any of the compound groups is arranged in any of the lattices of the lattice space, followed by arranging a next compound group in the lattice space, a limited lattice space that is a space created by eliminating, from the lattice space, undesirable regions for the next compound group to be arranged, is generated by the limiting unit 13. For example, a space limiting parameter L (L < n) representing a size of a diamond lattice space is set (S103), and a collection of lattice points to which i-numbered amino acid residue is move under the limit of the space limiting parameter L is determined as $V_i$ (S104).

$V_i$ that is the space for the i-numbered amino acid residue is represented by the following formula.

$$V_i = \bigcup_{r \in J} S_r$$

In the formula above, i = {1, 2, 3, ......n}.

When the space limiting parameter L is an even number, and i < L:

- J = {1, 3, .....i} in case of an odd numbered (i = odd number) amino acid residue.
- J = {2, 4, .....i} in case of an even numbered (i = even number) amino acid residue.

When the space limiting parameter L is an even number and i > L:

- J = {1, 3, .....L-1} in case of an odd numbered (i = odd number) amino acid residue.
- J = {2, 4, .....L} in case of an even numbered (i = even number) amino acid residue.

When the space limiting parameter L is an odd number and i < L:

- J = {1, 3, .....i} in case of an odd numbered (i = odd number) amino acid residue.
- J = {2, 4, .....i} in case of an even numbered (i = even number) amino acid residue.

When the space limiting parameter L is an odd number and i > L:

- J = {1, 3, .....L} in case of an odd numbered (i = odd number) amino acid residue.
- J = {2, 4, .....L-1} in case of an even numbered (i = even number) amino acid residue.

As described above, a space to which an amino acid residue is arranged is determined.

<Step S105>

Next, the assigning unit 14 is configured to assign a bit to each of the lattice points to which a plurality of compound groups are arranged in the limited lattice space. Specifically, special information is assigned to each of bits $X_1$ to $X_n$ (S105). As illustrated in FIGs. 11B to 11E, specifically, a bit expressing presence of an amino acid residue in that position as 1 and absence of an amino acid residue as 0 is assigned with respect to a space to which each of amino acid residues is arranged. Note that, in FIGs. 11A to 11C, a plurality of $X_i$ are assigned to amino acid residues 2 to 4, but in reality one bit $X_i$ is assigned to one amino acid residue 1.

<Step S106>

Next, $H_{one}$, $H_{conn}$, $H_{olap}$, and $H_{pair}$ are set and an Ising model obtained through conversion based on restriction conditions related to each lattice point is created (S106).

Setting of $H_{one}$, $H_{conn}$, $H_{olap}$, and $H_{pair}$ is performed in each of a $H_{one}$ generating unit 15A, a $H_{conn}$ generating unit 15B, a $H_{olap}$ generating unit 15C, and a $H_{pair}$ generating unit 15D of the H generating unit 15.

In the diamond encoding method, the entire energy can be expressed as follows.

$$E(x) = H = H_{one} + H_{conn} + H_{olap} + H_{pair}$$

In the formula above, $H_{one}$ is a restriction that there is only one from each of first to n-numbered amino acids.

$H_{conn}$ is a restriction that the first to n-numbered amino acids are all linked with one another.

$H_{olap}$ is a restriction that the first to n-numbered amino acids are not overlapped with one another.

$H_{pair}$ is a restriction representing an interaction between amino acids.

One example of each restriction is as follows.

Note that, in FIGs. 12 to 15 described below, $X_1$ is a position to which an amino acid residue of No. 1 can be arranged.

$X_2$ to $X_5$ are positions to which an amino acid residue of No. 2 can be arranged.

$X_6$ to $X_{13}$ are positions to which an amino acid residue of No. 3 can be arranged.

$X_{14}$ to $X_{29}$ are positions to which an amino acid residue of No. 4 can be arranged.

One example of $H_{one}$ is presented below.

$$H_{one} = \lambda_{one} \sum_{i=0}^{N-1} \sum_{x_a, x_b, \in Q_i, a<b} x_a x_b$$

In the function above, $X_a$ and $X_b$ may be 1 or 0. Specifically, $H_{one}$ is a function that energy increases when any two or more of $X_2$, $X_3$, $X_4$, and $X_5$ are 1, because only one of $X_2$, $X_3$, $X_4$, and $X_5$ is 1 in FIG. 12, and is a term of penalty and becomes 0 when only one of $X_2$, $X_3$, $X_4$, and $X_5$ is 1.

Note that, in the function above, $\lambda_{one}$ is a weighting coefficient.

One example of $H_{conn}$ is presented below.

$$H_{conn} = \lambda_{conn} \left(N - 1 - \sum_{i=0}^{N-1} \sum_{x_d \in Q_i} \sum_{x_u \in \eta(x_d) \cap Q_{i+1}} x_d x_u\right)$$

In the function above, $X_d$ and $X_u$ may be 1 or 0. Specifically, $H_{conn}$ is a formula that energy decreases as long as $X_{13}$, $X_6$, or $X_7$ is 1 when $X_2$ is 1 in FIG. 13, and is a penalty term and becomes 0 when all of the amino acid residues are linked with one another.

Note that, in the function above, $\lambda_{conn}$ is a weighting coefficient. For example, the relationship of $\lambda_{one} > \lambda_{conn}$ is satisfied.

One example of $H_{olap}$ is presented below.

$$H_{olap} = \lambda_{olap} \sum_{v \in V} \sum_{x_a, x_b, \in \theta(v), a<b} x_a x_b$$

In the function above, $X_a$ and $X_b$ are 1 or 0. Specifically, $H_{olap}$ is a term generating a penalty when $X_{14}$ is 1 with

$X_2$ being 1 in FIG. 14.

Note that, in the function above, $\lambda_{olap}$ is a weighting coefficient.

One example of $H_{pair}$ is presented below.

$$H_{pair} = \frac{1}{2}\sum_{i=0}^{N-1}\sum_{x_a \in Q_i}\sum_{x_b \in \eta(x_a)} P_{\omega(x_a)\omega(x_b)} x_a x_b$$

In the function above, $X_a$ and $X_b$ may be 1 or 0. Specifically, $H_{pair}$ is a function that energy decreases due to interaction $P_{\omega(x1)\omega(x15)}$ between the amino acid residue of $X_1$ and the amino acid residue of $X_{15}$ when $X_{15}$ is 1 with $X_1$ being 1 in FIGs. 15A and 15B. The interaction $P_{\omega(x1)\omega(x15)}$ is determined by a combination of two amino acid residues. For example, the interaction $P_{\omega(x1)\omega(x15)}$ is determined with reference to Miyazawa-Jernigan (MJ) matrix.

Next, H is calculated by synthesizing $H_{one}$, $H_{conn}$, $H_{olap}$, and $H_{pair}$ by the synthesizing unit 15E.

Next, a weighting coefficient ($\lambda_{one}$, $\lambda_{conn}$, and $\lambda_{olap}$) of each functions above is extracted by the weight extracting unit 16.

Next, a weight file corresponding to the extracted weight coefficient is created by the weight file creating unit 17. For example, the weight file is a matrix. In case of $2X_1X_2 + 4X_2X_3$, for example, the weight file is a matrix file as illustrated in FIG. 16.

The following energy formula of the Ising model can be expressed by using the created weight file.

$$E(x) = -\sum_{\langle i,j \rangle} W_{ij} x_i x_j - \sum_i b_i x_i$$

In the function above, the states $X_i$ and $X_j$ may be 0 or 1, where 0 means absence and 1 means presence. Wij that is a first term of the right side is a weighting coefficient.

The first term of the right side is the integration of the product of the state of two neuron circuits and the weighting value for all selectable combinations of two neuron circuits from the whole neuron circuits without any omission or overlap.

Moreover, the second term of the right side is the integration of the product of the bias value and state of each of the whole neuron circuits. $b_i$ is a bias value of the i-numbered neuron circuit.

<Step S107>

Next, the arithmetic unit 18 (annealing machine) executes a ground state search of the Ising model converted based on the restriction conditions related to each of the lattice points according to simulated annealing to thereby calculate the minimum energy of the Ising model (S107).

The arithmetic unit 18 (annealing machine) may be any of a quantum annealing machine, a semiconductor annealing machine using a semiconductor technology, or simulated annealing executed by software using a central processing unit (CPU) or a graphics processing unit (GPU), if the computer for use is a computer employing an annealing system for performing a ground state search of an energy function represented by the Ising model.

One example of simulated annealing and the arithmetic unit 18 (annealing machine) will be described below.

Simulated annealing (SA) is a kind of Monte Carlo methods, and a method for stochastically determining using a random numerical value. In the description below, a problem for minimizing a value of an evaluation function to be optimized is taken as an example, and the value of the evaluation function is called energy. In case of maximization, a plus or minus sign of the evaluation function may be changed.

Starting with an initial state where one discrete value is assigned to each variable, a state that is close to the initial state (e.g., a state where only one variable is changed) is selected from the current state (combinations of values of the variables), and then state transition thereof is studied. An energy change for the state transition is calculated, and whether the state transition is adapted to change the state or the original state is retained without adapting the state transition is determined stochastically depending on the calculated value. When the adaption probability of a case where energy reduces is selected to be larger than the adaption probability of a case where energy increases, the state change occurs in the tendency that the energy reduces on average, and it is expected that the state transits to an appropriate state over time. Then, ultimately, it is possible to obtain an approximation solution that gives energy close to an optimum solution or an optimum value. If the case where energy reduces is adopted deterministically and the case where energy increases is not adapted, the energy change is in the state of weakly decreasing with respect to time, but the change will stop once the local solution is reached. Since there are a large number of local solutions in the discrete optimization problem as described above, it is most likely that the state is trapped by a local solution that is not very close to an optimum value. Accordingly, it is important to stochastically determine whether to adapt.

It is proved in the simulated annealing that the state reaches the optimum solution with the limit of infinite time (the number of iteration) when the adaptation (tolerance) probability of the state transition is determined as follows. (1) With respect to an energy change (energy decrease) value (-ΔE) along with the state transition, acceptance probability p of the state transition is determined by any of the following functions f ().

$$p(\Delta E,T)=f(-\Delta E/T) \qquad \text{(Formula 1-1)}$$

$$f_{metro}(x)=\min(1,e^{x}) \quad \text{(Metropolis method)} \qquad \text{(Formula 1-2)}$$

$$f_{Gibbs}(x)=\frac{1}{1+e^{-x}} \quad \text{(Gibbs method)} \qquad \text{(Formula 1-3)}$$

In the formula above, T is a parameter called a temperature value, which is changed as follows. (2) The temperature value T is logarithmically decreased relative to the number of iteration t as represented by the following formula.

$$T=\frac{T_0\log(c)}{\log(t+c)} \qquad \text{(Formula 2)}$$

In the formula above, $T_0$ is an initial temperature value, and is desired to be sufficiently large depending on a problem.

In the case where acceptance probability represented by the formula of (1) is used, once the state reaches a steady state after sufficient iterations, occupancy probability of each state follows the Boltzmann distribution for a thermal equilibrium state in thermodynamics.

As the temperature is gradually lowered from a high temperature, occupancy probability of a low energy state increases. Therefore, a low energy state is supposed to be obtained when the temperature is sufficiently reduced. The state as described above is very similar to a state change occurred when a material is developed. Therefore, the method described above is called simulated annealing. The stochastic occurrence of the state transition of energy increase is equivalent to thermal excitation in physics.

An optimizing device (arithmetic unit 18) for performing simulated annealing is illustrated in FIG. 17. The descriptions below include a case where a plurality of candidates of state transitions are generated, but a transition candidate is generated one by one in the original basic simulated annealing.

An optimizing device 100 includes a state retaining unit 111 configured to retain a current state S (a plurality of state variable values). Moreover, the optimizing device 100 includes an energy calculating unit 112 configured to calculate an energy change value {-ΔEi} of each state transition when a state transition occurs from the current state S due to a change of any of the state variable values. Moreover, the optimizing device 100 includes a temperature controlling unit 113 configured to control a temperature value T and a transition controlling unit 114 configured to control a state transition.

The transition controlling unit 114 is configured to stochastically determine whether any of the state transitions is adapted or not according to the correlation between the energy change value {-ΔEi} and the thermal excitation energy based on the temperature value T, the energy change value {-ΔEi}, and the random numerical value.

The transition controlling unit 114 is further subdivided. The transition controlling unit 114 includes a candidate generating unit 114a configured to generate candidates of state transition, and a judging unit 114b configured to stochastically judge on each candidate whether the state transition is allowed or not based on the energy change value {-ΔEi} and temperature value T thereof. The transition controlling unit 114 further includes a transition determining unit 114c configured to determine the candidate to be adapted among the allowed candidates, and a random number generating unit 114d configured to generate probability variables.

An operation of one iteration is as follows. First, the candidate generating unit 114a generates one or more candidates (candidate number {Ni}) of state transition from the current state S retained in the state retaining unit 111 to the next state. The energy calculating unit 112 calculates an energy change value {-ΔEi} for each of state transitions listed as candidates using the current state S and the candidates of state transition. The judging unit 114b accepts the state transition with the acceptance probability of the formula of (1) above according to the energy change value {-ΔEi} of each state transition using the temperature value T generated by the temperature controlling unit 113 and the probability variable (random numerical value) generated by the random number generating unit 114d. Then, the judging unit 114b outputs acceptance or rejection {fi} of each state transition. In the case where there are a plurality of the accepted state transitions, the transition determining unit 114c randomly selects one of the accepted state transitions using the random numerical value. The transition determining unit 114c outputs the transition number N of the selected state transition and acceptance or rejection of the transition f. In the case where there is the accepted state transition, the value of the state variable stored in the state retaining unit 111 is updated according to the adapted state transition.

The iteration described above is started from an initial state and repeated with decreasing the temperature value by the temperature controlling unit 113. When the finishing judgement conditions, such as reaching the certain number of iterations, or the energy being dropped below a certain value, are satisfied, the operation is completed. The answer output by the optimizing device 110 is the state at the time of the finish.

FIG. 18 is a block diagram of a circuit level of a structural example of arithmetic part used for a transition controlling unit, particularly a judging unit, in typical simulated annealing where a candidate is generated one by one.

A transition controlling unit 114 includes a random number generator 114b1, a selector 114b2, a noise table 114b3, a multiplier 114b4, and a comparator 114b5.

The selector 114b2 is configured to select the value corresponding to the transition number N that is a random numerical value generated by the random number generator 114b1 among the energy change values {-ΔEi} calculated for candidates of each state transition, and then output the value.

Functions of the noise table 114b3 will be described later. As the noise table 114b3, for example, a memory, such as a random access memory (RAM), and a flash memory, can be used.

The multiplier 114b4 outputs a product (corresponding to the above-described thermal excitation energy) obtained by multiplying the value output by the noise table 114b3 with the temperature value T.

The comparator 114b5 outputs, as transition acceptance or rejection f, a comparison result obtained by comparing the product result output by the multiplier 114b4 and the energy change value -ΔE selected by the selector 114b2.

The transition controlling unit 114 illustrated in FIG. 18 basically has the above-mentioned functions as they are, but a mechanism for accepting state transition with the acceptance probability represented by the formula (1) has not yet been described. Therefore, the mechanism will be supplementary described.

The circuit that outputs 1 with the acceptance probability p and 0 with (1-p) has two inputs A and B, can be realized by inputting the acceptance probability p to the input A of the comparator and a uniform random number having the value in the interval [0, 1] to the input B of the comparator where the comparator outputs 1 when A > B and outputs 0 when A < B. Accordingly, the above-described function can be realized by inputting the value of the acceptance probability p calculated from the energy change value and the temperature value T using the formula of (1) to the input A of the comparator.

Specifically, the above-described function can be realized with the circuit that outputs 1 when f(ΔE/T) is larger than u, where f is the function represented by the formula of (1) and u is a uniform random number having the value of the interval [0, 1].

The circuit may be as it is, but the same function can be also realized by performing the following deformation. The magnitude relationship of two numbers does not change when the same monotone increasing function is given the two numbers. Therefore, output does not change even when the same monotone increasing function is gives two inputs of the comparator. It can be understood that a circuit outputting 1 when -ΔE/T is larger than $f^{-1}(u)$ is acceptable when an inverse function $f^{-1}$ of f is used as the monotone increasing function. Since the temperature value T is a positive value, moreover, a circuit outputting 1 when -ΔE is larger than $Tf^{-1}(u)$ is acceptable. The noise table 114b3 in FIG. 18 is a conversion table for realizing the inverse function $f^{-1}(u)$, and a table for outputting a value of the following function with respect to an input of discretized interval [0, 1].

$$f_{metro}^{-1}(u) = \log(u) \qquad \text{(Formula 3-1)}$$

$$f_{Gibbs}^{-1}(u) = \log\left(\frac{u}{1-u}\right) \qquad \text{(Formula 3-2)}$$

The transition controlling unit 114 also includes a latch configured to retain judgement results etc., a state machine configured to generate timing thereof, etc., but the above-mentioned units are omitted in FIG. 18 in order to simplify the illustration.

FIG. 19 illustrates an operation flow of the transition controlling unit 114. The operation flow includes a step for selecting one state transition as a candidate (S0001), a step for determining acceptance or rejection of the state transition with comparing a product of the energy change value of the state transition, temperature value, and random numerical value (S0002), and a step for adapting the state transition if the state transition is acceptable and rejecting if the state transition is not acceptable (S0003).

<Step S108>

Next, as a confirmation whether the limited lattice space is set to have a sufficient space, the judging unit 19 judges whether any of the compound groups assigned to the lattice points is arranged on the outermost edge of the limited lattice space (S108).

Typically, the judgement is performed on a conformation of a protein having the minimum energy calculated.

During the judging, the judgement is performed on whether any of the compound groups excluding the compound group arranged first and the compound group arranged last is arranged on the outermost edge of the limited lattice space among the compound groups assigned to the lattice points. This is because the compound group arranged first is not typically arranged on the outermost edge. Moreover, this is because the arrangement of the compound group is not limited by the outermost edge as there is no more compound group to be arranged next even when the compound group arranged last is arranged on the outermost edge.

<Step S109>

When the judging unit 19 judges that any of the compound groups assigned to the lattice points is not arranged on the outermost edge of the limited lattice space, it is judged that a sufficient space of the limited lattice space is set, and a calculation result related to conformation of a protein having the calculated minimum energy is output (S109).

The calculation result is output from the outputting unit 21. The result may be output as a protein conformation diagram or may be output as coordinate information of each amino acid residue constituting a protein.

<Step S110>

When the judging unit 19 judges that any of the compound groups assigned to the lattice points is arranged on the outermost edge of the limited lattice space, meanwhile, it is judged that a sufficient space of the limited lattice space is not set and hence the limited lattice space is expanded. Therefore, expansion information K is added to the space limiting parameter L (S110). Then, assignment of bits to the lattice points included in the limited lattice space after the expansion and calculation of the minimum energy of the Ising model are performed (S104 to S107).

During this step, the controlling unit 20 causes the limiting unit 13 to execute expansion of the limited lattice space.

Moreover, the controlling unit 20 causes the assigning unit 14 to execute assignment of a bit to each lattice point included in the limited lattice space after the expansion.

Moreover, the controlling unit 20 causes the arithmetic unit 18 to execute calculation of the minimum energy of the Ising model.

When the limited lattice space is expanded, the limited lattice space is not typically expanded to the original lattice space.

For example, the embodiment of the expansion may be an embodiment where the limited lattice space is uniformly expanded by the predetermined number of lattice points towards the outside of the outermost edge of the limited lattice space, and may be an embodiment where only a lattice space surrounding the compound group arranged on the outermost edge of the limited lattice space is expanded.

Moreover, the controlling unit 20 preferably does not change the already assigned bits to the lattice points of the compound group judged as being arranged on the outermost edge of the limited lattice space and the compound group arranged the earlier than the compound group judged as being arranged on the outermost edge. In the case where there are a plurality of compound groups judged as being arranged on the outermost edge of the limited lattice space, the "compound group judged as being arranged on the outermost edge of the limited lattice space" is preferably the compound group arranged the earliest among the compound groups judged as being arranged on the outermost edge of the limited lattice space.

Moreover, more preferred is that a difference (n - M) between the order (n) of the arrangement of a compound group arranged at last, and the order (M) of arrangement of the compound group judged as being arranged on the outermost edge of the limited lattice space be considered in expansion information, and the controlling unit 20 be configured to cause the limiting unit 13 to execute expansion of the limited lattice space based on the expansion information in a manner that the limited lattice space is expanded smaller when the difference (n - M) is small than when the difference (n - M) is large.

One of the above-described embodiments may be performed or the above-described embodiments may be performed in combination. Examples thereof will be described in Examples 1 to 6 below.

<Example 1>

An example of expansion of a limited lattice space will be described with reference to drawings.

FIG. 20A illustrates an example where amino acid residues are arranged in a diamond lattice space. In FIG. 20A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 20A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

FIG. 20B illustrates an example where the diamond lattice space of FIG. 20A is expanded by one layer of the lattice points towards the outside of the outer shell (i.e., an example where the radius r is expanded to the radius r + 1). The limited lattice space is expanded by further adding one lattice point to the outside of the outer shell of the diamond lattice space of FIG. 20A.

The expansion is an embodiment where the limited lattice space is uniformly expanded by the predetermined number from the lattice number towards the outside of the outermost edge.

For example, the expansion above can be performed according to the expansion information K.

<Example 2>

When compound groups are sequentially arranged, arrangement of the compound groups is not restricted by the outermost edge of the limited lattice space until the compound group is arranged on the outermost edge of the limited lattice space.

Therefore, preferred in view of reduction in a calculation time is that, as well as expanding the limited lattice space with maintaining the arrangement of the compound groups until a compound group is arranged on the outermost edge of the limited lattice space, assignment of bits to the lattice points included in the limited lattice space after the expansion and calculation of the minimum energy of the Ising model be executed.

In the case where assignment of bits to the lattice points included in the limited lattice space after the expansion and calculation of the minimum energy of the Ising model are executed as well as expanding the limited lattice space, it is preferred that bits already assigned to the lattice points of the compound group judged as being arranged on the outermost edge of the limited lattice space and the compound group arranged the earlier than the compound group judged as being arranged on the outermost edge be not changed. In the case where a plurality of compound groups are judged as being arranged on the outermost edge of the limited lattice space, in Example 2, the "compound group judged as being arranged on the outermost edge of the limited lattice space" is preferably the compound group arranged the earliest among the compound groups judged as being arranged on the outermost edge.

The examples above will be described with a flowchart and drawings.

FIG. 21 illustrates another structural example of the disclosed device for searching a compound. The device for searching a compound 10B illustrated in FIG. 21 is identical to the device for searching a compound 10A illustrated in FIG. 6 in that the device for searching a compound 10B includes a compound group number counting unit 11, a defining unit 12, a limiting unit 13, an assigning unit 14, a H generating unit 15, a weight extracting unit 16, a weight file creating unit 17, an arithmetic unit 18, a judging unit 19, a controlling unit 20, and an outputting unit 21. However, the device for searching a compound 10B illustrated in FIG. 21 is different from the device for searching a compound 10A illustrated in FIG. 6 in that the generating unit 15 of the device for searching a compound 10B includes a $H_{fix}$ generating unit 15F.

The flowchart of FIG. 22 is a flowchart in which Step S111 is added to the flowchart of FIG. 7.

FIG. 23A is an example where amino acid residues are arranged in a diamond lattice space.

In FIG. 23A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 23A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

As illustrated in FIG. 23B, for example, the diamond lattice space of FIG. 23A is expanded by a layer of the lattice points (i.e., the radius r is expanded to the radius r + 1). Then, Steps S104 to S107 are performed using the expanded diamond lattice space. At the time when Ising model is created, a constraint term $H_{fix}$ , which is configured not to change a bit already assigned, is added to the No 4 amino acid residue judged as being arranged on the outermost edge of the limited lattice space and the Nos. 1 to 3 amino acid residues having the smaller number of the arrangement than the No. 4 amino acid residue (Sill). The constraint term $H_{fix}$ is created by a $H_{fix}$ generating unit 15F under the control of the controlling unit 20. When the Ising model is created, the bits already assigned to the lattice points of the No. 4 amino acid residue arranged on the outermost edge of the limited lattice space and the Nos. 1 to 3 amino acid residues arranged the earlier than the No. 4 amino acid residue can be unchanged by adding the constraint term $H_{fix}$. This can be illustrated as in FIG. 23C. FIG. 23C is a diagram illustrating a state where the Nos. 1 to 4 amino acid residues are fixed in the expanded diamond lattice space of FIG. 23B.

For example, the constraint term $H_{fix}$ is represented by the following formula.

$$H_{fix} = \lambda_{fix} \sum_{i=1}^{M} (1 - x_{A_i})$$

In the function above, $X_{Ai}$ is an address to be fixed (not changed), and i is the sequence number of the amino acid residues.

In the function above, $\lambda_{fix}$ is a weighting coefficient.

E(x) to which the constraint term $H_{fix}$ is added can be expressed as follows.

$$E(x) = H = H_{one} + H_{conn} + H_{olap} + H_{pair} + H_{fix}$$

The constraint term $H_{fix}$ is prioritized than other constraint terms by making $\lambda_{fix}$ of the constraint term $H_{fix}$ sufficiently larger than the weighting coefficient than other constraint terms, and therefore $X_{Ai}$ is fixed to 1.

<Example 3>

An embodiment of the expansion is preferably an embodiment where only the lattice space surrounding the compound group arranged on the outermost edge of the limited lattice space is expanded. There is a less possibility that an amino acid residue is arranged even when the lattice space other than the space surrounding the compound group arranged on the outermost edge of the limited lattice space is expanded. Therefore, the expanding range can be maintained to an appropriate range by using an embodiment where only the lattice space surrounding the compound group arranged on the outermost edge of the limited lattice space.

To expand only the lattice space surrounding the compound group arranged on the outermost edge of the limited lattice space may be referred to as "giving the expansion directionality" hereinafter.

An embodiment of Example 3 is an embodiment where directionality is given to the expansion in the embodiment of Example 2.

An embodiment of Example 3 will be described with reference to drawings.

FIG. 24A illustrates an example where amino acid residues are arranged in a diamond lattice space.

In FIG. 24A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 24A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

As illustrated in FIG. 24B, for example, only the lattice space surrounding the amino acid residue arranged on the outermost edge of the limited lattice space (No. 4 amino acid residue) in the diamond lattice space of FIG. 24Ais expanded. Steps S104 to S107 are performed using the expanded diamond lattice space. When the Ising model is created, the already assigned bits assigned to the lattice points of the No. 4 amino acid residue judged as being arranged on the outermost edge of the limited lattice space and the Nos. 1 to 3 amino acid residues arranged the earlier than the No. 4 amino acid residue are not changed (FIG. 24C).

<Example 4>

In the case where the sequence number of the compound group arranged on the outermost edge of the limited lattice space is large, the number of compound groups (remaining compound groups) having the larger sequence number than the compound group arranged on the outermost edge is small. In the case where the sequence number of the compound group arranged on the outermost edge of the limited lattice space is small, on the other hand, on the other hand, the number of compound groups (remaining compound groups) having the larger sequence number than the compound group arranged on the outermost edge is large. In the case where the number of the remaining compound groups is large, appropriate expansion may not be performed unless a range of the expansion is made large. In the case where the number of the remaining compound groups is small, on the other hand, most of the expanded range may be pointless.

Therefore, a range of expansion of the limited lattice space is preferably made large when the sequence number of the compound group arranged on the outermost edge of the limited lattice space is small. A range of expansion of the limited lattice space is preferably made small when the sequence number of the compound group arranged on the outermost edge of the limited lattice space is large.

Specifically, it is preferred that the limited lattice space is preferably expanded in a manner that the limited lattice space be expanded smaller when the difference (n - M) is small than when the difference (n - M) is large, considering in the expansion information K the difference (n - M) between the order (n) of the arrangement of the compound group arranged last and the order (M) of the arrangement of the compound group judged as being arranged on the outermost edge of the limited lattice space.

The above-described embodiment may be referred to as an "amino acid residue number dependency" of the expansion range hereinafter.

The embodiment of Example 4 is an embodiment where the amino acid residue number dependency is given to the expansion range in the embodiment of Example 1.

An embodiment of Example 4 will be described with reference to drawings.

FIG. 25A illustrates an example where amino acid residues are arranged in a diamond lattice space.

In FIG. 25A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 25A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

When the expansion is performed, a range K of the expansion (the number of layers of the lattice points) is determined based on the following function.

$$K=\mathrm{roundup}((n-M)/2)$$

In the equation above, "round up" is a function for rounding up after the decimal point.

In case of n = 7 and M = 4, for example, (7 - 4)/2 = 1.5 and K = 2. As illustrated in FIG. 25B, the outer shell is expanded by two layers of the lattice point towards the outside of the outer shell in the diamond lattice space of FIG. 25A (i.e., the radius r is expanded to the radius r + 2). Then, Steps S104 to S107 are performed using the expanded diamond lattice space.

<Example 5>

Example 5 is an embodiment where Example 4 and Example 2 are combined.

The embodiment of Example 5 will be described with reference to drawings.

FIG. 26A illustrates an example where amino acid residues are arranged in a diamond lattice space.

In FIG. 26A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 26A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

When the expansion is performed, a range K of the expansion (the number of layers of the lattice points) is determined based on the following function.

$$K=\mathrm{roundup}((n-M)/2)$$

In the equation above, "round up" is a function for rounding up after the decimal point.

In case of n = 7 and M = 4, for example, (7 - 4)/2 = 1.5 and K = 2. As illustrated in FIG. 26B, the outer shell is expanded by two layers of the lattice point towards the outside of the outer shell in the diamond lattice space of FIG. 26A (i.e., the radius r is expanded to the radius r + 2). Then, Steps S104 to S107 are performed using the expanded diamond lattice space. When the Ising model is created, the already assigned bits assigned to the lattice points of the No. 4 amino acid residue judged as being arranged on the outermost edge of the limited lattice space and the Nos. 1 to 3 amino acid residues arranged the earlier than the No. 4 amino acid residue are not changed (FIG. 26C).

<Example 6>

Example 6 is an embodiment where Example 4 and Example 3 are combined.

The embodiment of Example 6 will be described with reference to drawings.

FIG. 27A illustrates an example where amino acid residues are arranged in a diamond lattice space.

In FIG. 27A, in the case where the number (n) of the amino acid residues is 7 (n = 7), a space limiting parameter L is set to 4 (L = 4), 7 amino acid residues are arranged in a diamond lattice space having a radius r of 4 (r = 4).

In FIG. 27A, the No. 4 amino acid residue is arranged on the outermost edge of the diamond lattice space.

Therefore, it is judged by the judgment performed in Step S108 that any of compound groups assigned to lattice points is arranged on the outermost edge of the limited lattice space and expansion of the diamond lattice space is performed.

When the expansion is performed, a range K of the expansion (the number of layers of lattice points) is determined based on the following function with giving the directionality to the range of the expansion.

$$K=\mathrm{roundup}((n-M)/2)+1$$

In the equation above, "round up" is a function for rounding up after the decimal point.

In case of n = 7 and M = 4, for example, (7 - 4)/2 = 1.5 and K = 3. As illustrated in FIG. 27B, only the lattice space surrounding the amino acid residue (No. 4 amino acid residue) arranged on the outermost edge of the limited lattice space is expanded by three layers in the diamond lattice space of FIG. 27A. Then, Steps S104 to S107 are performed using the expanded diamond lattice space. When the Ising model is created, the already assigned bits assigned to the lattice points of the No. 4 amino acid residue judged as being arranged on the outermost edge of the limited lattice space and the Nos. 1 to 3 amino acid residues arranged the earlier than the No. 4 amino acid residue are not changed (FIG. 27C).

Examples 1 to 6 are described above, and can be summarized in FIG. 28.

Note that, the phrase "Is it automatically set?" in FIG. 28 corresponds to whether there is the amino acid residue number dependency described above.

Moreover, the conventional method in FIG. 28 means that a lattice space is not limited in the diamond encoding method.

Moreover, Referential Example in FIG. 28 means a case where a lattice space is limited but whether the limited lattice space is expanded is not judged and the limited lattice space is not expanded.

Note that, the device for searching a compound 10A of FIG. 6 is an example where the arithmetic unit 18 and the limiting unit 13 are arranged in the identical space, but the device for searching a compound may be a device in which the arithmetic unit 18 and the limiting unit 13 are spatially separated as in the device for searching a compound 10C illustrated in FIG. 29.

Next, a modified example of the steps S101 to S107 of the flowchart of FIG. 7 will be described.

FIG. 30 is a flowchart of a modified example.

In the flowchart of FIG. 30, the step S201 corresponds to the step S101 of the flowchart of FIG. 7, the step S202 corresponds to the step S102, the step S204 corresponds to the step S104, the step S205 corresponds to the step S105, the step S206 corresponds to the step S106, and the step S207 corresponds to the step S107.

Therefore, the descriptions are given with focusing on the limiting unit 13 and the step S203.

By setting the maximum number M (straight chain number limiting parameter M) of amino acid residues aligned in a straight chain (S203), the limiting unit 13 generates a limited lattice space obtained by removing, from the lattice space, undesirable regions for the next compound group to be arranged, in the case where any of a plurality of compound groups is arranged in any lattice of the lattice space, followed by arranging the next compound group in the lattice space.

As described earlier, amino acid residues are typically rarely aligned in a straight chain due to interaction between the amino acid residues.

Therefore, the number of arithmetic bits or quantum bits can be suppressed by setting the maximum number M (straight chain number limiting parameter M) of amino acid residues aligned in a straight chain, and eliminating regions where the amino acid residues are not be disposed under the restrictions above to thereby generate a limited lattice space. Naturally, M is smaller than the number (n) of amino acid residues (M < n).

For example, when the straight chain number limiting parameter M is set to 5, as illustrated in FIG. 31, the number of amino acid residues aligned in a straight chain is 5 as the maximum number.

When the straight chain number limiting parameter M is set, the limited lattice space increases as the number of the amino acid residues increases as illustrated in FIG. 32. Specifically, the maximum lattice space K is determined by the following formula when the straight chain limiting parameter M is used for the amino acid residues in the number of n.

$$K = \left\lfloor \frac{n - M}{M} \right\rfloor \times (M - 2) + MAX(n\ mod[M] - 2, 0) + M$$

A space limiting parameter L (L < n) may be used in combination for generation of a limited lattice space. In this case, it is preferable that L ≤ K be satisfied.

FIG. 33 is a flowchart of another modified example.

In the flowchart of FIG. 33, the step S301 corresponds to the step S201 of the flowchart of FIG. 21, the step S302 corresponds to the step S202, the step S303 corresponds to the step S203, the step S305 corresponds to the step S204, the step S306 corresponds to the step S205, the step S307 corresponds to the step S206, and the step S308 corresponds to the step S207.

Therefore, descriptions are given with focusing on the limiting unit 13 and the step S304.

In the case where any of a plurality of compound groups is arranged in any lattice of a lattice space followed by arranging a next compound group in the lattice space, the limiting unit 13 creates a limited lattice space, which is obtained by eliminating undesirable regions for the next compound group to be arranged from the lattice space, by setting the maximum number M (straight chain limiting parameter M) of amino acid residues aligned in a straight chain (S303), and moreover defining the maximum S(i) of the site to which i-numbered amino acid residue is moved (S304).

When the straight chain number limiting parameter M is used, a space radius r of each amino acid residue is for example as presented in Table 1 with M = 5 (K = 8), n = 11, and L = K.

Table 1

| Amino acid residue | 1 | 2 | 3 | 4 | 5 | 6 | 7 | 8 | 9 | 10 | 11 |
|---|---|---|---|---|---|---|---|---|---|---|---|
| r | 1 | 2 | 3 | 4 | 5 | 6 | 7 | 8 | 8 | 8 | 8 |
| Radius r' actually estimated | 1 | 2 | 3 | 4 | 5 | 4 | 5 | 6 | 7 | 8 | 7 |

The above-described example is visualized as in FIG. 34. Although the maximum space is identical, excess space is created and it can be understood that the $6^{th}$ or $7^{th}$ amino acid residue can be made the smaller space in reality.

Therefore, the straight chain limiting parameter M is added and a space parameter s(x) using the straight chain number limiting parameter s(x). As a result, the space can be limited as follows, and the number of bits can be suppressed without lowering accuracy.

$$V_i = \bigcup_{r \in J} S_r$$

i = {1, 2, 3, ......n}

$$s(x) = \left\lfloor \frac{x-1}{M} \right\rfloor \times (M-2) + \left((x-1) mod M\right) + 1$$

When the space limiting parameter L is an even number, and i < L:

- J = {s(1), s(3), .....S(i)} in case of an odd numbered (i = odd number) amino acid residue.
- J = {s(2), s(4), .....S(i)} in case of an even numbered (i = even number) amino acid residue.

When the space limiting parameter L is an even number, and i > L:

- J = {s(2), s(4), S(L - 1)} in case of an odd numbered (i = odd number) amino acid residue.
- J = {s(2), s(4), .....S(L)} in case of an even numbered (i = even number) amino acid residue.

When the space limiting parameter L is an odd number, and i < L:

- J = {s(1), s(3), .....S(i)} in case of an odd numbered (i = odd number) amino acid residue.
- J = {s(2), s(4), .....S(i)} in case of an even numbered (i=even number) amino acid residue.

When the space limiting parameter L is an odd number, and i > L:

- J = {s(2), s(4), .....S(L)} in case of an odd numbered (i = odd number) amino acid residue.
- J = {s(2), s(4), S(L - 1)}} in case of an even numbered (i = even number) amino acid residue.

Regarding the technology of referential examples, an embodiment where a straight chain number limiting parameter is not set is determined as Referential Example 1, a modified example for describing using FIG. 30 is determined as Referential Example 2, and a modified example for describing unit FIG. 33 is determined as Referential Example 3. In each Referential Examples, a change of the number of bits used when the parameter is determined as follows is illustrated in FIG. 35.

- Referential Example 1: L = 15
- Referential Example 2: L = 15, M = 5
- Referential Example 3: L = 15, M = 5
- Comparative Example 1: No restriction

It was confirmed in all of Examples that the number of bits used could be significantly reduced compared to Comparative Example 1 where no restriction was given, and a compound having relatively a large scale of a problem (e.g., a protein) could be used as a target of a search.

According to the referential examples above, however, the arrangement of the compound groups is limited by the outermost edge of the limited lattice space if the limited lattice space is two narrow. As a result, an appropriate conformation may not be obtained.

Therefore, the number of bits can be appropriately suppressed, for example, by combining any of the referential examples with Examples 1 to 6, compared with the conventional technology.

When a lattice space is not limited, for example, the number of bits used for amino acid residues in the number of n is represented by the following formula.

$$\sum_{i=1}^{n} \left\{ 2\left( \sum_{j=1}^{i-1} j^2 \right) + i^2 - mod(i,2) \right\} + 1$$

When the limit of the lattice space (L: space limiting parameter) is set to L = n - 1 and the radium of the space to be expanded (K: expansion information) is set to K = 1, in the case where the limited lattice space is expanded with directionality, for example, the directional increase is 3 bits when the amino acid residue arranged on the outermost edge is positioned on the outermost plane of the limited lattice space, is 4 bits when the amino acid residue arranged on the outermost edge is positioned on the outermost side of the limited lattice space, and is 5 bits when the amino acid residue arranged on the outermost edge is positioned on the outermost apex of the limited lattice space. Therefore, the number of bits used for expanding the limited lattice space with directionality can be represented by the following formula when the limit of the lattice space (L: space limiting parameter) is set to L = n - 1 and the radius of the space to be expanded (K: expansion information) is set to K = 1.

$$\sum_{i=1}^{n}\left\{2\left(\sum_{j=1}^{i-1} j^2\right)+i^2-mod(i,2)\right\}-\sum_{i=1}^{n-1}\left\{2\left(\sum_{j=1}^{i-1} j^2\right)+i^2-mod(i,2)\right\}+[3\ or\ 4\ or\ 5]$$

An example where 11 amino acid residues are arranged as in FIG. 36 will be described hereinafter. Note that, the numbers in FIG. 36 are numbers representing the positions of the lattice points in the diamond lattice space.

When the lattice space for the 11 amino acid residues is not limited (in the case of n = 11), the number of bits used is 2,921 bits.

In case of the arrangement as in FIG. 36, it is appropriate that the limited lattice space to be L = 5, and the number of bits used is 153 bits, when the lattice space is limited, but whether the limited lattice space is expanded is not judged and the limited lattice space is not expanded as in the referential examples.

In case of the arrangement as in FIG. 36, however, the limited lattice space may be set to L = 4 and may be expanded with directionality by K = 1, i.e., 3 bits or 5 bits. In this case, the number of bits used is 69 bits (in case of r = 4) + 3 bits or 5 bits = 72 bits or 74 bits. Therefore, 81 bits (= 153 bits - 72 bits) or 79 bits (= 153 bits - 74 bits) can be reduced compared to the case where the limited lattice space is simply set to L=5.

Note that, such a reduction effect becomes the larger as the number of the amino acid residues is the larger.

## Claims

**1.** A device (10) for searching a compound, comprising:

a defining unit (12) configured to define a lattice space that is a collection of lattice points where a plurality of compound groups are sequentially arranged;
a limiting unit (13) configured to, in a case where any of the compound groups is arranged in any of the lattice points of the lattice space, followed by arranging a next compound group in any of the rest of the lattice points, generate a limited lattice space that is a space created by eliminating, from the lattice space, regions for the next compound group to be arranged, by setting the maximum number of compound groups aligned in a straight chain and eliminating regions where the compound groups are not disposed;
an assigning unit (14) configured to assign a bit to each of the lattice points, to which the compound groups can be arranged, in the limited lattice space;
an arithmetic unit (18) configured to perform a ground state search on an Ising model obtained through conversion based on restriction conditions related to each of the lattice points according to simulated annealing, to thereby calculate minimum energy of the Ising model;
a judging unit (19) configured to judge whether any of the compound groups assigned to the lattice points is arranged on outermost lattice points of the limited lattice space or not; and
a controlling unit (20) configured to cause the limiting unit (13) to execute expansion of the limited lattice space by further adding a lattice point to the outside of the outermost lattice points, cause the assigning unit (14) to execute assignment of the bits to the lattice points included in the limited lattice space after the expansion, and cause the arithmetic unit (18) to execute calculation of the minimum energy of the Ising model, in a case where the judging unit (19) judges that any of the compound groups assigned to the lattice points is arranged on the outermost lattice points of the limited lattice space,
wherein the device is a device for searching the compound, in which the plurality of the compound groups are linked with one another.

**2.** The device (10) according to claim 1,
wherein the judging unit (19) is configured to judge whether any of the compound groups excluding the compound group arranged first and the compound group arranged last is arranged on the outermost lattice points of the limited lattice space among the compound groups assigned to the lattice points.

**3.** The device (10) according to claim 1 or 2,

wherein the controlling unit is configured to cause the limiting unit to execute expansion of the limited lattice space based on expansion information, wherein the expansion information is the number of layers of the lattice points to be added in the expansion.

**4.** The device according to any one of claims 1 to 3,
wherein the controlling unit (20) is configured not to change the bits already assigned to the lattice points of the compound group judged as being arranged on the outermost lattice points of the limited lattice space and the compound groups arranged earlier than the compound group judged as being arranged on the outermost lattice points of the limited lattice space.

**5.** The device (10) according to any one of claims 1 to 4,
wherein the controlling unit (20) is configured to expand only a lattice space surrounding the compound group arranged on the outermost lattice points of the limited lattice space when the controlling unit (20) causes the limiting unit (13) to execute expansion of the limited lattice space.

**6.** The device according to any one of claims 3 to 5,

wherein the expansion information considers a difference (n - M) between the order (n) of the arrangement of a compound group arranged last, and the order (M) of arrangement of the compound group judged as being arranged on the outermost lattice points of the limited lattice space, and
the controlling unit (20) is configured to cause the limiting unit (13) to execute expansion of the limited lattice space based on the expansion information in a manner that the limited lattice space is expanded less when the difference (n - M) is smaller than when the difference (n - M) is larger.

**7.** The device according to any one of claims 1 to 6,
wherein the compound groups are amino acid residues.

**8.** The device according to claim 7,
wherein the compound is a protein.

**9.** A method for searching a compound, the method comprising:

defining a lattice space that is a collection of lattice points where a plurality of compound groups are sequentially arranged;
in a case where any of the compound groups is arranged in any of the lattice points of the lattice space, followed by arranging a next compound group in the lattice space, generating a limited lattice space that is a space created by eliminating, from the lattice space, regions for the next compound group to be arranged, by setting the maximum number of compound groups aligned in a straight chain and eliminating regions where the compound groups are not disposed;
assigning a bit to each of the lattice points, to which the compound groups can be arranged, in the limited lattice space;
performing a ground state search on an Ising model obtained through conversion based on restriction conditions related to each of the lattice points according to simulated annealing, to thereby calculate minimum energy of the Ising model;
judging whether any of the compound groups assigned to the lattice points is arranged on the outermost lattice points of the limited lattice space or not; and
executing expansion of the limited lattice space by further adding a lattice point to the outside of the outermost lattice points, assignment of the bits to the lattice points included in the limited lattice space after the expansion, and calculation of the minimum energy of an Ising model, in a case where it is judged that any of the compound groups assigned to the lattice points is arranged on the outermost lattice points of the limited lattice space,
wherein the method is a method for allowing a computer to search the compound in which the plurality of the compound groups are linked with one another.

**10.** A program for causing a computer to execute a method for searching a compound, the method comprising:

defining a lattice space that is a collection of lattice points where a plurality of compound groups are sequentially arranged;
in a case where any of the compound groups is arranged in any of the lattice points of the lattice space, followed by arranging a next compound group in the lattice space, generating a limited lattice space that is a space created by eliminating, from the lattice space, regions for the next compound group to be arranged, by setting the maximum number of compound groups aligned in a straight chain and eliminating regions where the compound groups are not disposed;
assigning a bit to each of the lattice points, to which the compound groups can be arranged, in the limited lattice space;
performing a ground state search on an Ising model obtained through conversion based on restriction conditions related to each of the lattice points according to simulated annealing, to thereby calculate minimum energy of the Ising model;
judging whether any of the compound groups assigned to the lattice points is arranged on the outermost lattice points of the limited lattice space or not; and
executing expansion of the limited lattice space by further adding a lattice point to the outside of the outermost lattice points, assignment of the bits to the lattice points included in the limited lattice space after the expansion, and calculation of the minimum energy of an Ising model, in a case where it is judged that any of the compound groups assigned to the lattice points is arranged on the outermost lattice points of the limited lattice space,
wherein the method is a method for searching the compound in which the plurality of the compound groups are linked with one another.

## Patentansprüche

1. Vorrichtung (10) zum Suchen einer Verbindung, umfassend:

eine Definitionseinheit (12), die dazu eingerichtet ist, einen Gitterraum zu definieren, der eine Sammlung von Gitterpunkten ist, an denen eine Vielzahl von Verbindungsgruppen sequentiell angeordnet ist;
eine Begrenzungseinheit (13), die dazu eingerichtet ist, in einem Fall, in dem eine der Verbindungsgruppen an einem der Gitterpunkte des Gitterraums angeordnet ist, gefolgt von einer Anordnung einer nächsten Verbindungsgruppe an einem der übrigen Gitterpunkte, einen begrenzten Gitterraum zu erzeugen, der ein Raum ist, der geschaffen wird durch Eliminieren von Bereichen aus dem Gitterraum für die nächste anzuordnende Verbindungsgruppe durch Einstellen der maximalen Anzahl von Verbindungsgruppen, die in einer geraden Kette angeordnet sind, und Eliminieren von Bereichen, in denen die Verbindungsgruppen nicht angeordnet sind;
eine Zuordnungseinheit (14), die dazu eingerichtet ist, in dem begrenzten Gitterraum jedem der Gitterpunkte, an denen die Verbindungsgruppen angeordnet werden können, ein Bit zuzuordnen;
eine Arithmetikeinheit (18), die dazu eingerichtet ist, eine Grundzustandssuche in einem Ising-Modell durchzuführen, das durch Transformieren basierend auf Beschränkungsbedingungen bezüglich jedem der Gitterpunkte gemäß simuliertem Abkühlen erhalten wird, um dadurch die minimale Energie des Ising-Modells zu berechnen;
eine Beurteilungseinheit (19), die dazu eingerichtet ist, zu beurteilen, ob eine der den Gitterpunkten zugeordneten Verbindungsgruppen an den äußersten Gitterpunkten des begrenzten Gitterraums angeordnet ist oder nicht; und
eine Steuereinheit (20), die dazu eingerichtet ist, die Begrenzungseinheit (13) zu veranlassen, eine Erweiterung des begrenzten Gitterraums auszuführen durch weiteres Hinzufügen eines Gitterpunkts außerhalb der äußersten Gitterpunkte, die Zuordnungseinheit (14) zu veranlassen, eine Zuordnung von Bits zu den Gitterpunkten, die in dem begrenzten Gitterraum nach der Erweiterung enthalten sind, auszuführen, und die Arithmetikeinheit (18) zu veranlassen, eine Berechnung der minimalen Energie des Ising-Modells auszuführen, in einem Fall, in dem die Beurteilungseinheit (19) beurteilt, dass einer der den Gitterpunkten zugeordnete Verbindungsgruppen an den äußersten Gitterpunkten des begrenzten Gitterraums angeordnet ist,
wobei die Vorrichtung eine Vorrichtung zum Suchen der Verbindung ist, in der die Vielzahl der Verbindungsgruppen miteinander verbunden sind.

2. Vorrichtung (10) nach Anspruch 1,
wobei die Beurteilungseinheit (19) dazu eingerichtet ist, zu beurteilen, ob eine der Verbindungsgruppen, mit Ausnahme der zuerst angeordneten Verbindungsgruppe und der zuletzt angeordneten Verbindungsgruppe, unter den den Gitterpunkten zugeordneten Verbindungsgruppen an den äußersten Gitterpunkten des begrenzten Gitterraums angeordnet ist.

3. Vorrichtung (10) nach Anspruch 1 oder 2,
wobei die Steuereinheit dazu eingerichtet ist, die Begrenzungseinheit zu veranlassen, eine Erweiterung des begrenzten Gitterraums auszuführen, basierend auf Erweiterungsinformationen, wobei die Erweiterungsinformationen die Anzahl von Schichten der Gitterpunkte sind, die bei der Erweiterung hinzuzufügen sind.

**4.** Vorrichtung nach einem der Ansprüche 1 bis 3,
wobei die Steuereinheit (20) dazu eingerichtet ist, die Bits nicht zu ändern, die bereits den Gitterpunkten der Verbindungsgruppe, für die beurteilt wurde, dass sie an den äußersten Gitterpunkten des begrenzten Gitterraums angeordnet ist, und der Verbindungsgruppen, die früher angeordnet wurden als die Verbindungsgruppe, für die beurteilt wurde, dass sie an den äußersten Gitterpunkten des begrenzten Gitterraums angeordnet ist, zugeordnet wurden.

**5.** Vorrichtung (10) nach einem der Ansprüche 1 bis 4,
wobei die Steuereinheit (20) dazu eingerichtet ist, wenn die Steuereinheit (20) die Begrenzungseinheit (13) veranlasst, eine Erweiterung des begrenzten Gitterraum auszuführen, nur einen Gitterraum zu erweitern, der die Verbindungsgruppe umgibt, die an den äußersten Gitterpunkten des begrenzten Gitterraums angeordnet ist.

**6.** Vorrichtung nach einem der Ansprüche 3 bis 5,

wobei die Erweiterungsinformationen eine Differenz (n - M) berücksichtigen zwischen der Reihenfolge (n) der Anordnung einer zuletzt angeordneten Verbindungsgruppe und der Reihenfolge (M) der Anordnung der Verbindungsgruppe, für die beurteilt wurde, dass sie an den äußersten Gitterpunkten des begrenzten Gitterraums angeordnet ist, und
die Steuereinheit (20) dazu eingerichtet ist, die Begrenzungseinheit (13) zu veranlassen, eine Erweiterung des begrenzten Gitterraums basierend auf den Erweiterungsinformationen auf eine Weise auszuführen, in der der begrenzte Gitterraum weniger erweitert wird, wenn die Differenz (n - M) kleiner ist, als wenn die Differenz (n - M) größer ist.

**7.** Vorrichtung nach einem der Ansprüche 1 bis 6,
wobei die Verbindungsgruppen Aminosäurereste sind.

**8.** Vorrichtung nach Anspruch 7,
wobei die Verbindung ein Protein ist.

**9.** Verfahren zum Suchen einer Verbindung, wobei das Verfahren umfasst:

Definieren eines Gitterraums, der eine Sammlung von Gitterpunkten ist, an denen eine Vielzahl von Verbindungsgruppen sequentiell angeordnet ist;
in einem Fall, in dem eine der Verbindungsgruppen an einem der Gitterpunkte des Gitterraums angeordnet ist, gefolgt von einer Anordnung einer nächsten Verbindungsgruppe in dem Gitterraum, Erzeugen eines begrenzten Gitterraums, der ein Raum ist, der geschaffen wird durch Eliminieren von Bereichen aus dem Gitterraum für die nächste anzuordnende Verbindungsgruppe durch Einstellen der maximalen Anzahl von Verbindungsgruppen, die in einer geraden Kette angeordnet sind, und Eliminieren von Bereichen, in denen die Verbindungsgruppen nicht angeordnet sind;
Zuordnen eines Bits zu jedem der Gitterpunkte in dem begrenzten Gitterraum, an denen die Verbindungsgruppen angeordnet werden können;
Durchführen einer Grundzustandssuche in einem Ising-Modell, das durch Transformieren basierend auf Beschränkungsbedingungen bezüglich jedem der Gitterpunkte gemäß simuliertem Abkühlen erhalten wird, um dadurch die minimale Energie des Ising-Modells zu berechnen;
Beurteilen, ob eine der den Gitterpunkten zugeordneten Verbindungsgruppen an den äußersten Gitterpunkten des begrenzten Gitterraums angeordnet ist oder nicht; und
Ausführen einer Erweiterung des begrenzten Gitterraums durch weiteres Hinzufügen eines Gitterpunkts außerhalb der äußersten Gitterpunkte, Zuordnen der Bits zu den Gitterpunkten, die in dem begrenzten Gitterraum nach der Erweiterung enthalten sind, und Berechnen der minimalen Energie des Ising-Modells, in einem Fall, in dem beurteilt wird, dass einer der den Gitterpunkten zugeordnete Verbindungsgruppen an den äußersten Gitterpunkten des begrenzten Gitterraums angeordnet ist,
wobei das Verfahren ein Verfahren ist, das es einem Computer ermöglicht, die Verbindung zu suchen, in der die Vielzahl der Verbindungsgruppen miteinander verbunden sind.

**10.** Programm zum Veranlassen eines Computers, ein Verfahren zum Suchen einer Verbindung auszuführen, wobei das Verfahren umfasst:

Definieren eines Gitterraums, der eine Sammlung von Gitterpunkten ist, an denen eine Vielzahl von Verbindungsgruppen sequentiell angeordnet ist;
in einem Fall, in dem eine der Verbindungsgruppen an einem der Gitterpunkte des Gitterraums angeordnet ist, gefolgt von einer Anordnung einer nächsten Verbindungsgruppe in dem Gitterraum, Erzeugen eines begrenzten Gitterraums, der ein Raum ist, der geschaffen wird durch Eliminieren von Bereichen aus dem Gitterraum für die nächste anzuordnende Verbindungsgruppe durch Einstellen der maximalen Anzahl von Verbindungsgruppen, die in einer geraden Kette angeordnet sind, und Eliminieren von Bereichen, in denen die Verbindungsgruppen nicht angeordnet sind;
Zuordnen eines Bits zu jedem der Gitterpunkte, an denen die Verbindungsgruppen in dem begrenzten Gitterraum angeordnet werden können;
Durchführen einer Grundzustandssuche in einem Ising-Modell, das durch Transformieren basierend auf Beschränkungsbedingungen bezüglich jedem der Gitterpunkte gemäß simuliertem Abkühlen erhalten wird, um dadurch die minimale Energie des Ising-Modells zu berechnen;
Beurteilen, ob eine der den Gitterpunkten zugeordneten Verbindungsgruppen an den äußersten Gitterpunkten des begrenzten Gitterraums angeordnet ist oder nicht; und
Ausführen einer Erweiterung des begrenzten Gitterraums durch weiteres Hinzufügen eines Gitterpunkts außerhalb der äußersten Gitterpunkte, Zuordnen der Bits zu den Gitterpunkten, die in dem begrenzten Gitterraum nach der Erweiterung enthalten sind, und Berechnen der minimalen Energie des Ising-Modells, in einem Fall, in dem beurteilt wird, dass einer der den Gitterpunkten zugeordnete Verbindungsgruppen an den äußersten Gitterpunkten des begrenzten Gitterraums angeordnet ist,
wobei das Verfahren ein Verfahren zum Suchen der Verbindung ist, in der die Vielzahl der Verbindungsgruppen miteinander verbunden sind.

## Revendications

**1.** Un dispositif (10) pour rechercher un composé, comprenant :

une unité de définition (12) configurée pour définir un espace de réseau qui est une collection de points de réseau où une pluralité de groupes de composés sont disposés séquentiellement ;
une unité de limitation (13) configurée pour, dans un cas où l'un quelconque des groupes de composés est disposé dans l'un quelconque des points de réseau de l'espace de réseau, suivi par la disposition d'un groupe de composés suivant dans l'un quelconque du reste des points de réseau,
générer un espace de réseau limité qui est un espace créé en éliminant, de l'espace de réseau, des régions pour le groupe de composés suivant à disposer, en fixant le nombre maximum de groupes de composés alignés en une chaîne droite et en éliminant les régions où les groupes de composés ne sont pas disposés ;
une unité d'attribution (14) configurée pour attribuer un bit à chacun des points de réseau, auxquels les groupes de composés peuvent être disposés, dans l'espace de réseau limité ;
une unité arithmétique (18) configurée pour effectuer une recherche d'état fondamental sur un modèle d'Ising obtenu par conversion sur la base de conditions de restriction liées à chacun des points de réseau selon un recuit simulé, pour calculer ainsi l'énergie minimale du modèle d'Ising ;
une unité de détermination (19) configurée pour déterminer si l'un quelconque des groupes de composés attribués aux points de réseau est disposé sur les points de réseau les plus extérieurs de l'espace de réseau limité ou non ; et
une unité de commande (20) configurée pour amener l'unité de limitation (13) à exécuter une expansion de l'espace de réseau limité en ajoutant en outre un point de réseau à l'extérieur des points de réseau les plus extérieurs, amener l'unité d'attribution (14) à exécuter une attribution des bits aux points de réseau inclus dans l'espace de réseau limité après l'expansion, et amener l'unité arithmétique (18) à exécuter le calcul de l'énergie minimale du modèle d'Ising, dans un cas où l'unité détermination (19) détermine que l'un quelconque des groupes de composés attribués aux points de réseau est disposé sur les points de réseau les plus extérieurs de l'espace de réseau limité,
dans lequel le dispositif est un dispositif pour rechercher le composé, dans lequel la pluralité des groupes de composés sont liés les uns aux autres.

**2.** Le dispositif (10) selon la revendication 1,
dans lequel l'unité détermination (19) est configurée pour déterminer si l'un quelconque des groupes de composés, à l'exclusion du groupe de composés disposé en premier et du groupe de composés disposé en dernier, est disposé sur les points de réseau les plus extérieurs de l'espace de réseau limité parmi les groupes de composés attribués aux points de réseau

3. Le dispositif (10) selon la revendication 1 ou 2, dans lequel l'unité de commande est configurée pour amener l'unité de limitation à exécuter une expansion de l'espace de réseau limité sur la base des informations d'expansion, dans lequel les informations d'expansion sont le nombre de couches des points de réseau à ajouter dans l'expansion.

4. Le dispositif selon l'une quelconque des revendications 1 à 3, dans lequel l'unité de commande (20) est configurée pour ne pas modifier les bits déjà attribués aux points de réseau du groupe de composés déterminé comme étant disposé sur les points de réseau les plus extérieurs de l'espace de réseau limité et les groupes de composés disposés avant le groupe de composés déterminé comme étant disposé sur les points de réseau les plus extérieurs de l'espace de réseau limité.

5. Le dispositif (10) selon l'une quelconque des revendications 1 à 4, dans lequel l'unité de commande (20) est configurée pour dilater uniquement un espace de réseau entourant le groupe de composés disposé sur les points de réseau les plus extérieurs de l'espace de réseau limité lorsque l'unité de commande (20) amène l'unité de limitation (13) à exécuter l'expansion de l'espace de réseau limité.

6. Le dispositif selon l'une quelconque des revendications 3 à 5,

   dans lequel les informations d'expansion considèrent une différence (n - M) entre l'ordre (n) de l'agencement d'un groupe de composés disposé en dernier, et l'ordre (M) d'agencement du groupe de composés déterminé comme étant disposé sur les points de réseau les plus extérieurs de l'espace de réseau limité, et
   l'unité de commande (20) est configurée pour amener l'unité de limitation (13) à exécuter l'expansion de l'espace de réseau limité sur la base des informations d'expansion d'une manière telle que l'espace de réseau limité est moins étendu lorsque la différence (n - M) est plus petite que lorsque la différence (n - M) est plus grande.

7. Le dispositif selon l'une quelconque des revendications 1 à 6, dans lequel les groupes de composés sont des résidus d'acides aminés.

8. Le dispositif selon la revendication 7, dans lequel le composé est une protéine.

9. Un procédé pour rechercher un composé, le procédé comprenant :

   la définition d'un espace de réseau qui est une collection de points de réseau où une pluralité de groupes de composés sont disposés séquentiellement ;
   dans un cas où l'un quelconque des groupes de composés est disposé dans l'un quelconque des points de réseau de l'espace de réseau, suivi par la disposition d'un groupe de composés suivant dans l'espace de réseau, la génération d'un espace de réseau limité qui est un espace créé en éliminant, de l'espace de réseau, des régions pour le groupe de composés suivant à disposer, en fixant le nombre maximum de groupes de composés alignés en une chaîne droite et en éliminant les régions où les groupes de composés ne sont pas disposés ;
   l'attribution d'un bit à chacun des points de réseau, auxquels les groupes de composés peuvent être disposés, dans l'espace de réseau limité,
   effectuer une recherche d'état fondamental sur un modèle d'Ising obtenu par conversion sur la base de conditions de restriction liées à chacun des points de réseau selon un recuit simulé, pour calculer ainsi l'énergie minimale du modèle d'Ising ;
   déterminer si l'un quelconque des groupes de composés attribués aux points de réseau est disposé sur les points de réseau les plus extérieurs de l'espace de réseau limité ou non ; et
   l'exécution de l'expansion de l'espace de réseau limité en ajoutant en outre un point de réseau à l'extérieur des points de réseau les plus extérieurs, en attribuant les bits aux points de réseau inclus dans l'espace de réseau limité après l'expansion, et le calcul de l'énergie minimale d'un modèle d'Ising, dans un cas où il est déterminé que l'un quelconque des groupes de composés attribués aux points de réseau est disposé sur les points de réseau les plus extérieurs de l'espace de réseau limité,
   dans lequel le procédé est un procédé pour permettre à un ordinateur de rechercher le composé dans lequel la pluralité des groupes de composés sont liés les uns aux autres.

**10.** Un programme pour amener un ordinateur à exécuter un procédé pour rechercher un composé, le procédé comprenant :

la définition d'un espace de réseau qui est une collection de points de réseau où une pluralité de groupes de composés sont disposés séquentiellement ;
dans un cas où l'un quelconque des groupes de composés est disposé dans l'un quelconque des points de réseau de l'espace de réseau, suivi par la disposition d'un groupe de composés suivant dans l'espace de réseau, la génération d'un espace de réseau limité qui est un espace créé en éliminant,
de l'espace de réseau, des régions pour le groupe de composés suivant à disposer, en fixant le nombre maximum de groupes de composés alignés en une chaîne droite et en éliminant les régions où les groupes de composés ne sont pas disposés ;
l'attribution d'un bit à chacun des points de réseau, auxquels les groupes de composés peuvent être disposés, dans l'espace de réseau limité,
effectuer une recherche d'état fondamental sur un modèle d'Ising obtenu par conversion sur la base de conditions de restriction liées à chacun des points de réseau selon un recuit simulé, pour calculer ainsi l'énergie minimale du modèle d'Ising ;
déterminer si l'un quelconque des groupes de composés attribués aux points de réseau est disposé sur les points de réseau les plus extérieurs de l'espace de réseau limité ou non; et
l'exécution de l'expansion de l'espace de réseau limité en ajoutant en outre un point de réseau à l'extérieur des points de réseau les plus extérieurs, en attribuant les bits aux points de réseau inclus dans l'espace de réseau limité après l'expansion, et le calcul de l'énergie minimale d'un modèle d'Ising, dans un cas où il est déterminé que l'un quelconque des groupes de composés attribués aux points de réseau est disposé sur les points de réseau les plus extérieurs de l'espace de réseau limité,
dans lequel le procédé est un procédé pour rechercher le composé dans lequel la pluralité des groupes du composé sont liés les uns aux autres.

FIG. 1

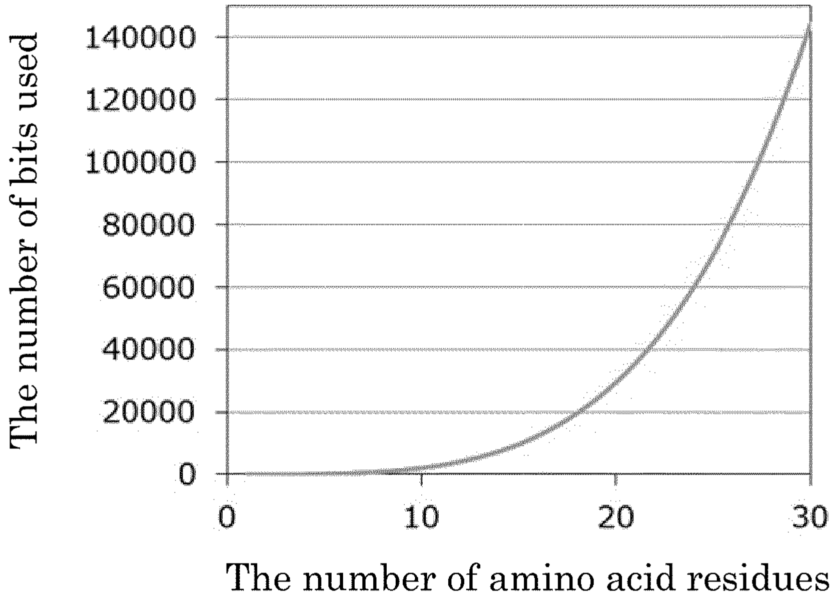


FIG. 2A

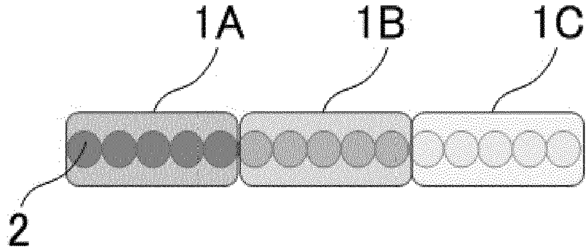


FIG. 2B

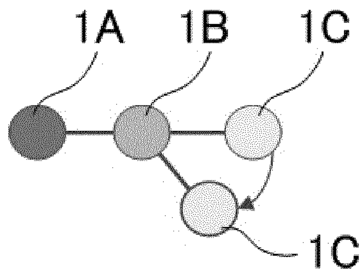

FIG. 2C

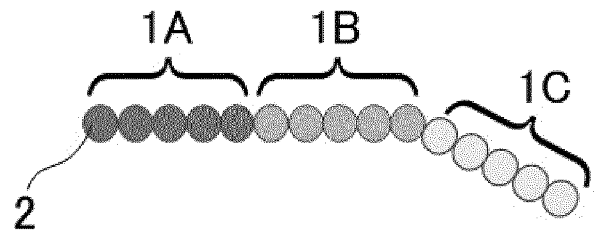


FIG. 3A

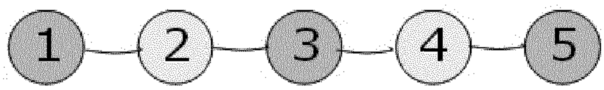


FIG. 3B

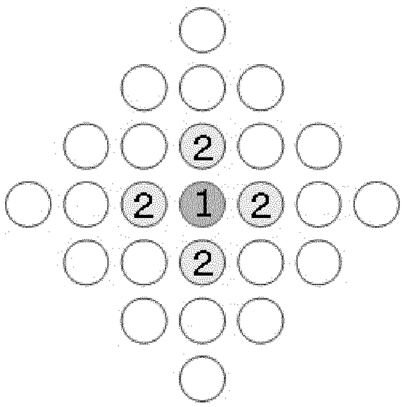


FIG. 3C

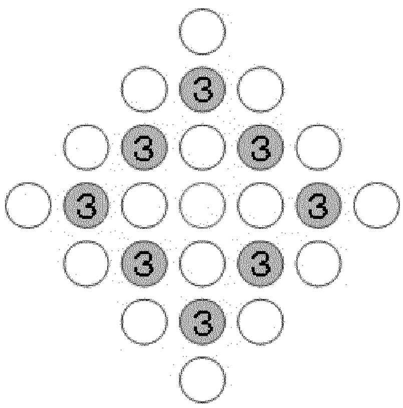

FIG. 3D

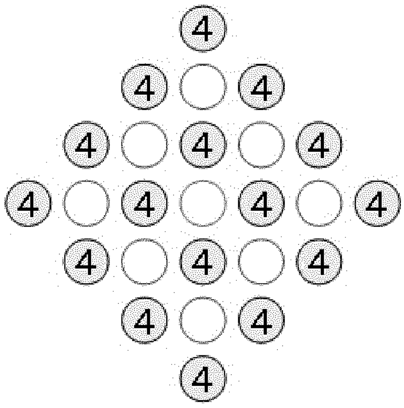

FIG. 3E

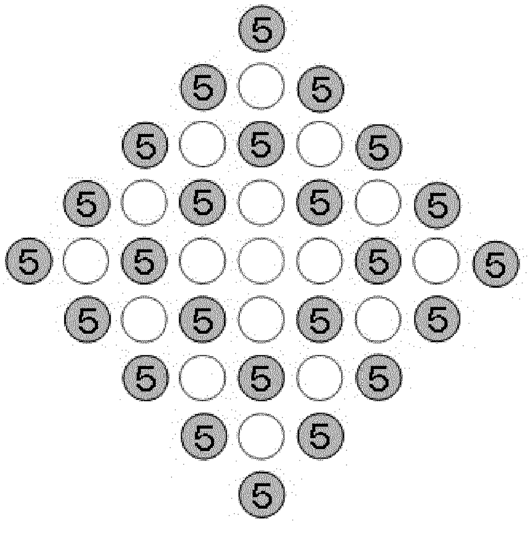

FIG. 4

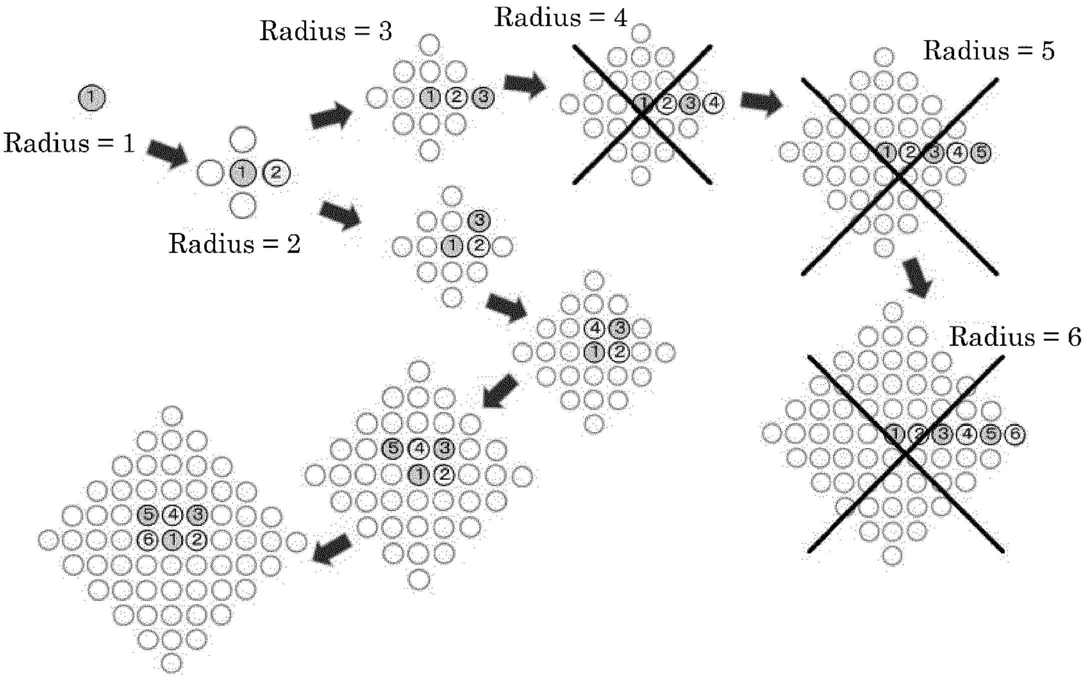


FIG. 5

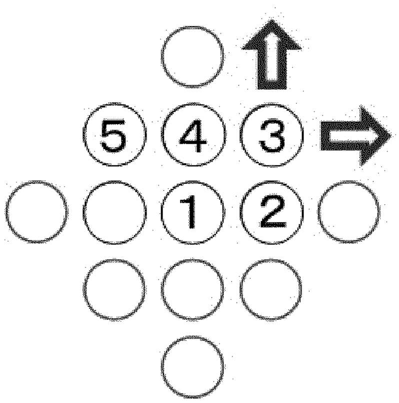

FIG. 6

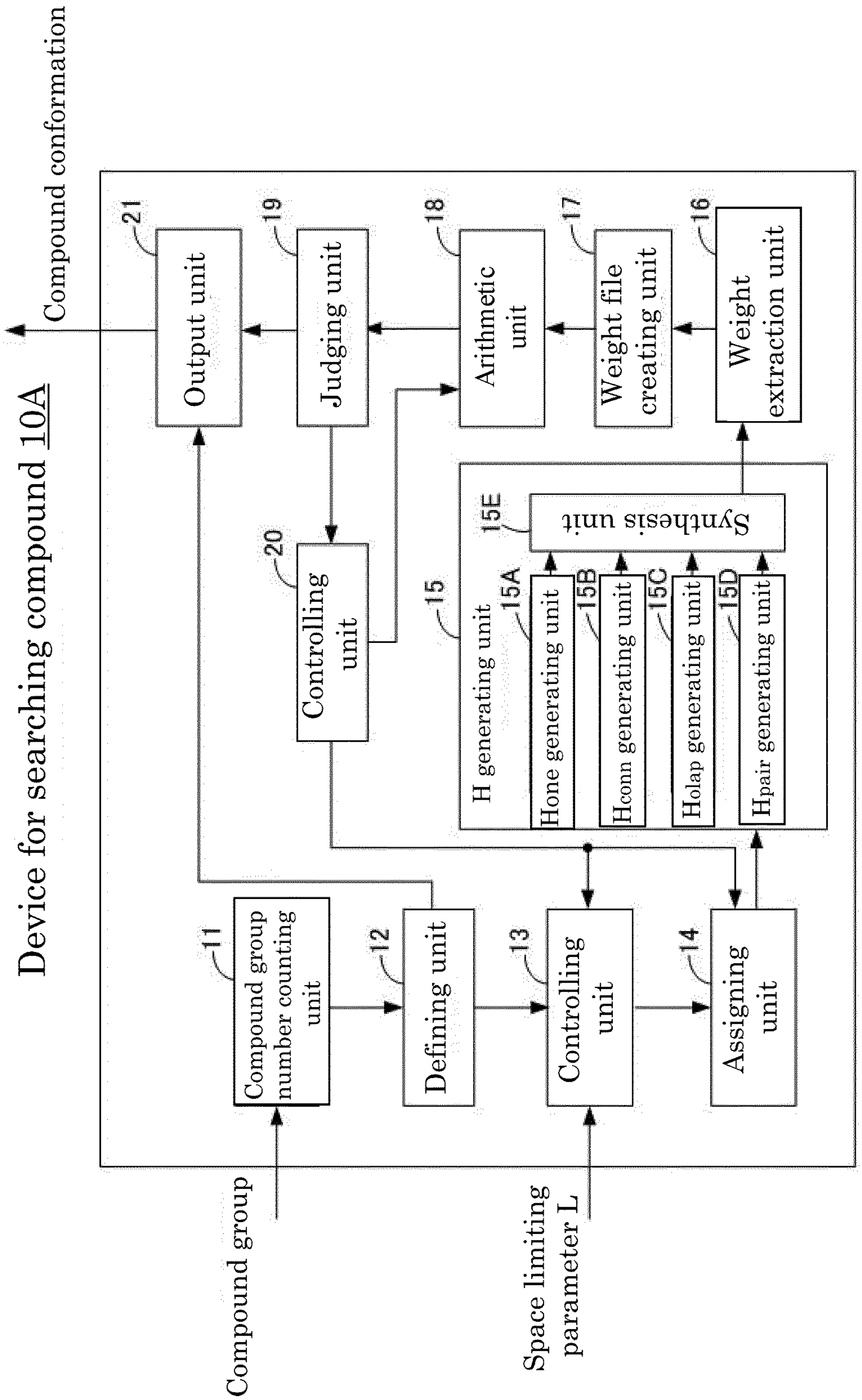
Device for searching compound 10A
Compound conformation
Compound group
Space limiting parameter L
11 Compound group number counting unit
12 Defining unit
13 Controlling unit
14 Assigning unit
15 H generating unit
15A Hone generating unit
15B Hconn generating unit
15C Holap generating unit
15D Hpair generating unit
15E Synthesis unit
16 Weight extraction unit
17 Weight file creating unit
18 Arithmetic unit
19 Judging unit
20 Controlling unit
21 Output unit

FIG. 7

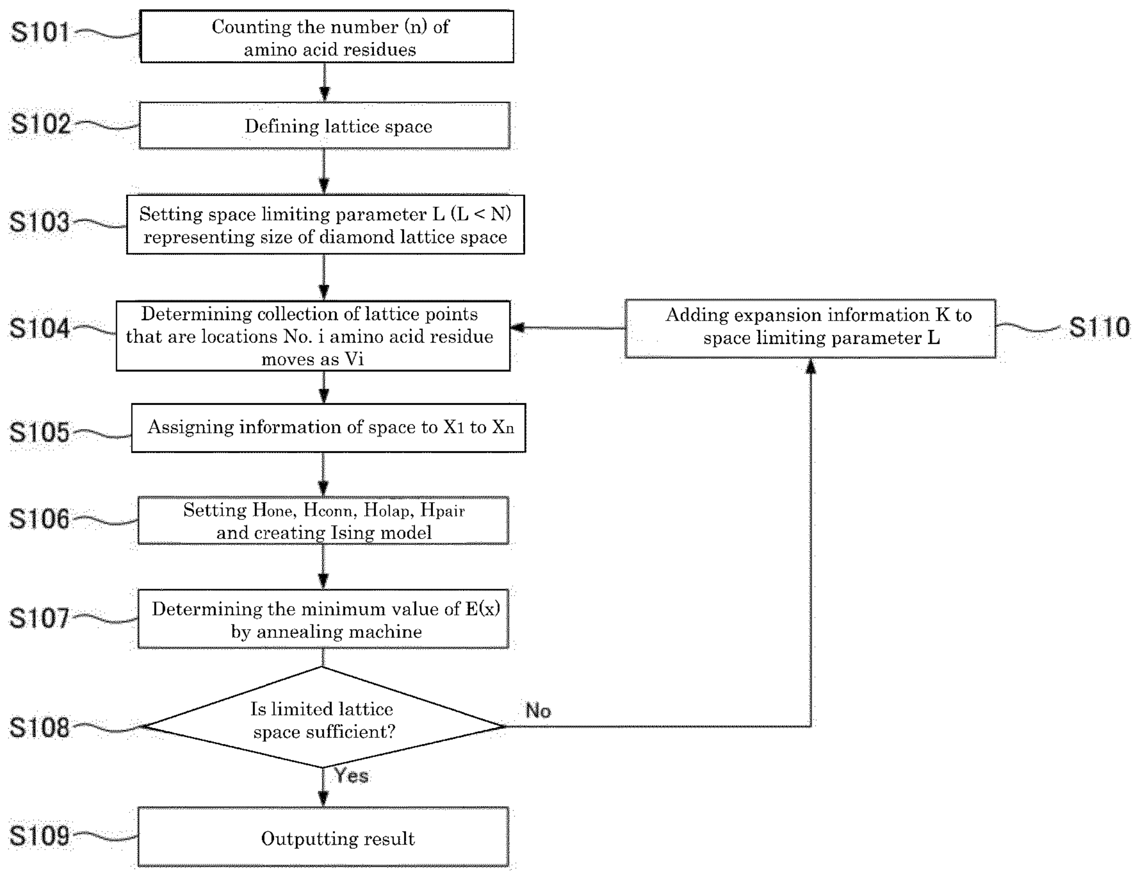
S101
Counting the number (n) of amino acid residues
S102
Defining lattice space
S103
Setting space limiting parameter L (L < N) representing size of diamond lattice space
S104
Determining collection of lattice points that are locations No. i amino acid residue moves as Vi
S105
Assigning information of space to X1 to Xn
S106
Setting Hone, Hconn, Holap, Hpair and creating Ising model
S107
Determining the minimum value of E(x) by annealing machine
S108
Is limited lattice space sufficient?
No
Yes
S109
Outputting result
S110
Adding expansion information K to space limiting parameter L

FIG. 8

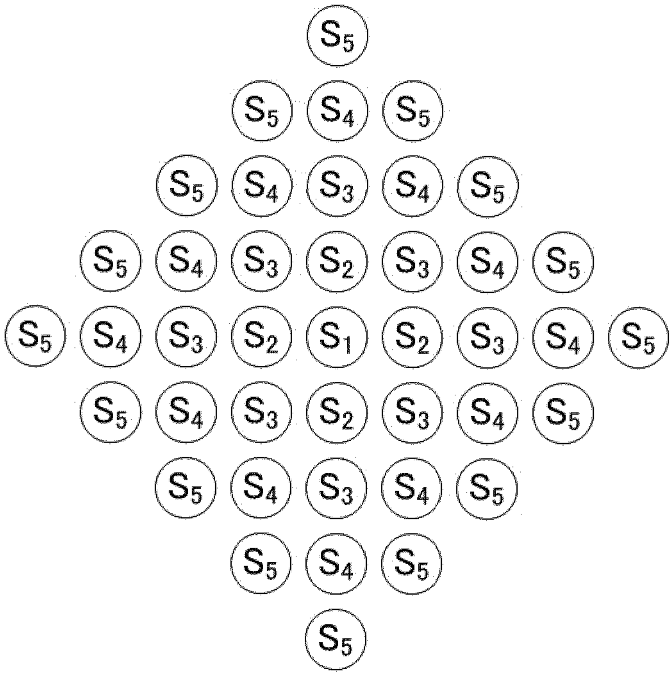
S5
S5 S4 S5
S5 S4 S3 S4 S5
S5 S4 S3 S2 S3 S4 S5
S5 S4 S3 S2 S1 S2 S3 S4 S5
S5 S4 S3 S2 S3 S4 S5
S5 S4 S3 S4 S5
S5 S4 S5
S5

FIG. 9A

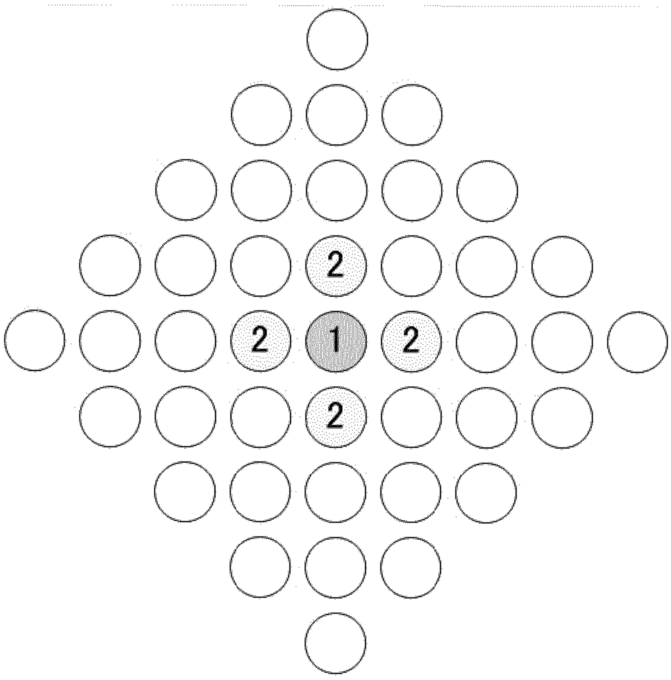
2
2 1 2
2

FIG. 9B

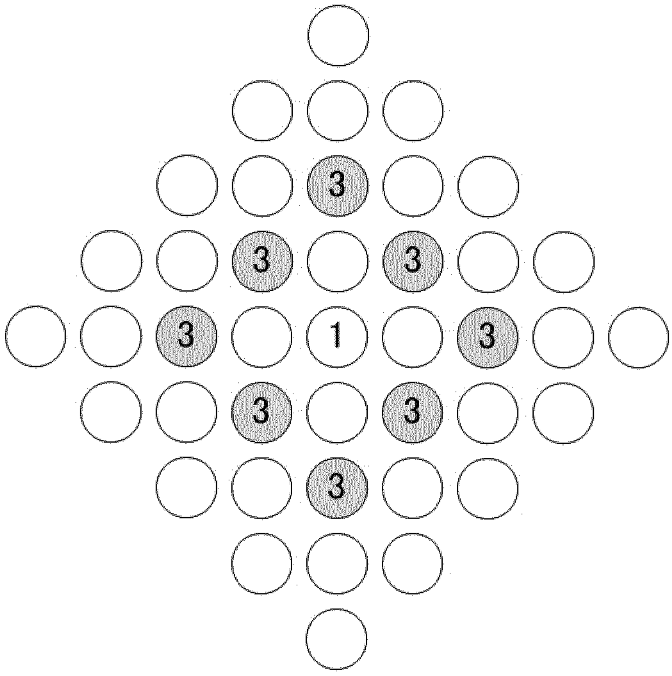


FIG. 9C

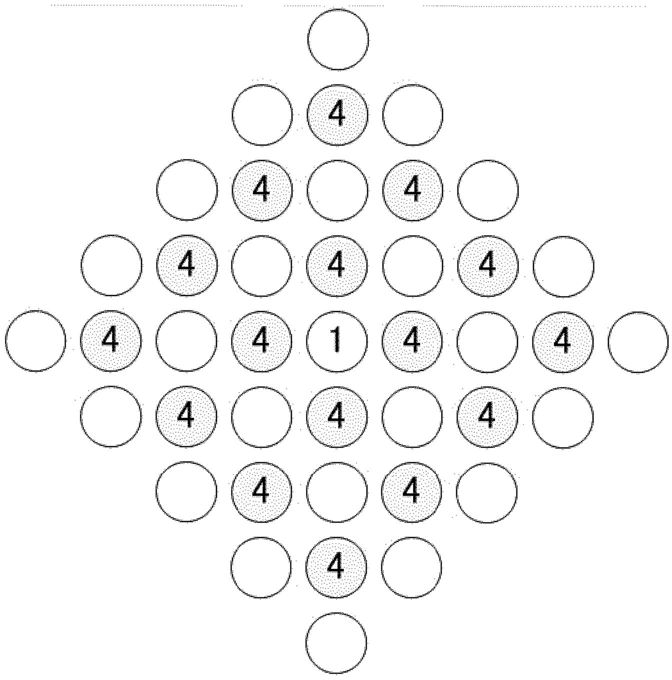

FIG. 9D

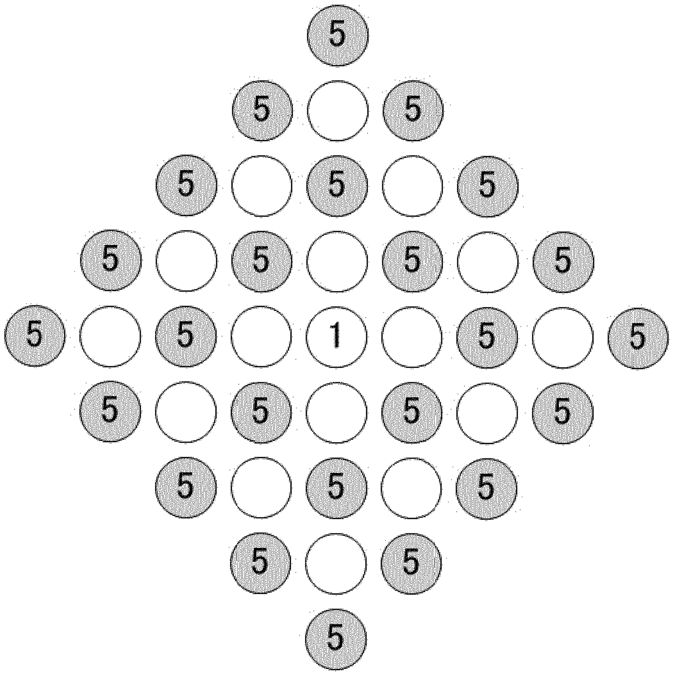
5
5
5
5
5
5
5
5
5
5
5
5
5
5
5
1
5
5
5
5
5
5
5
5
5
5
5
5
5

FIG. 10

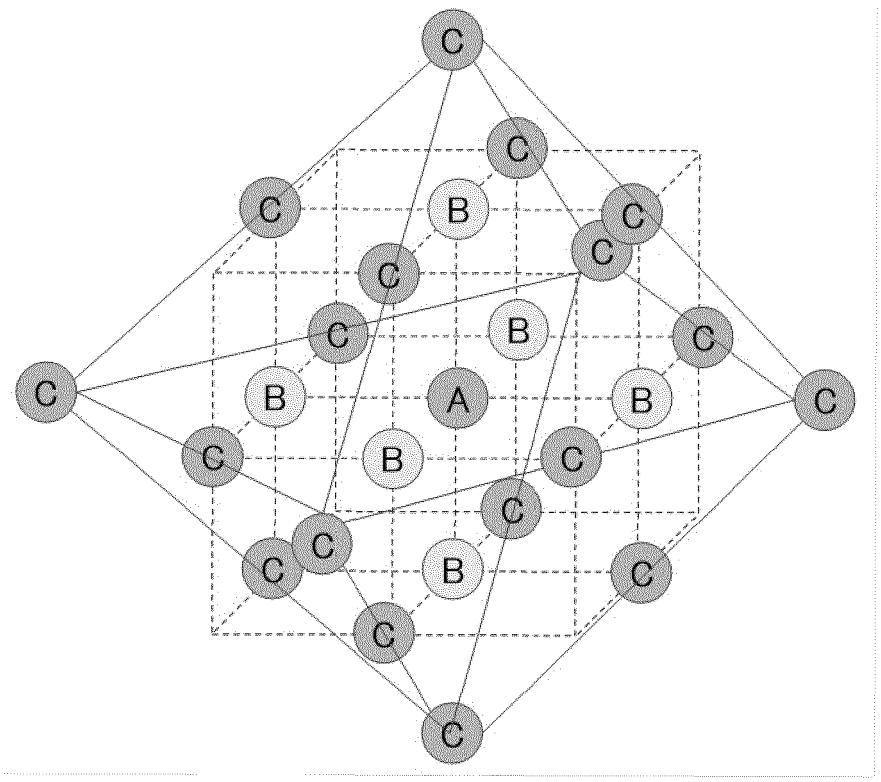
C
C
C
B
C
C
C
C
B
C
C
B
A
B
C
C
C
B
C
C
C
C
B
C
C
C

FIG. 11A

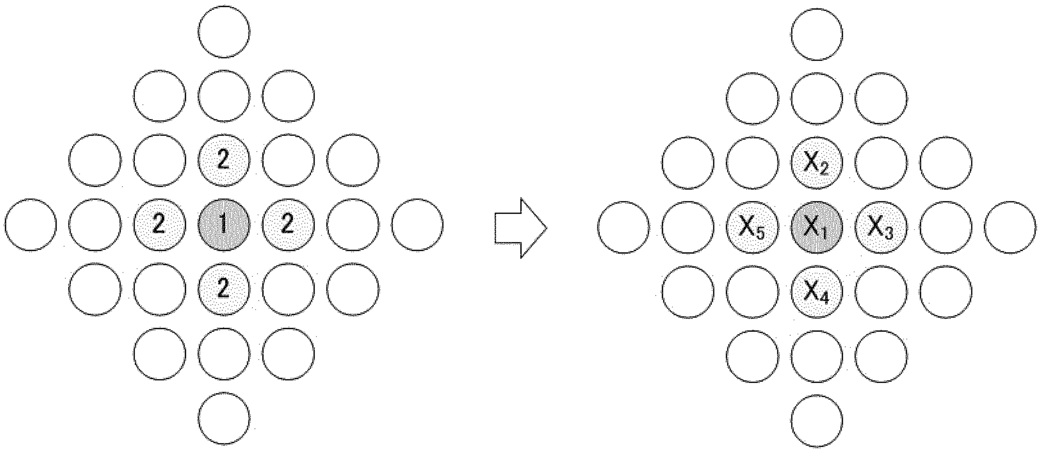


FIG. 11B

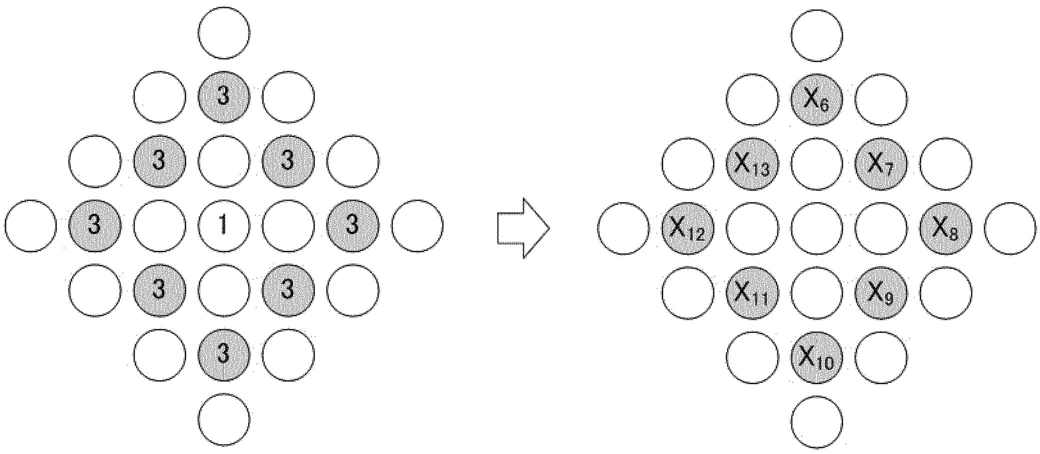


FIG. 11C

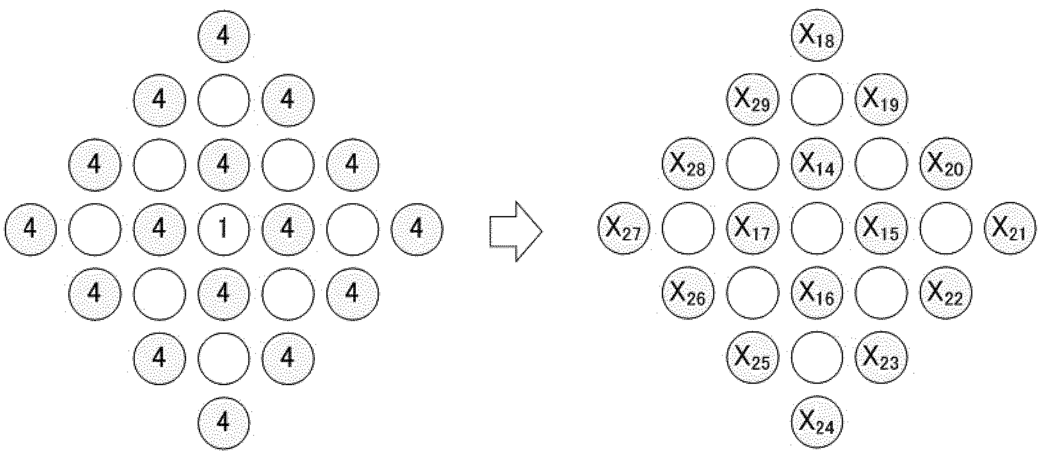

FIG. 12

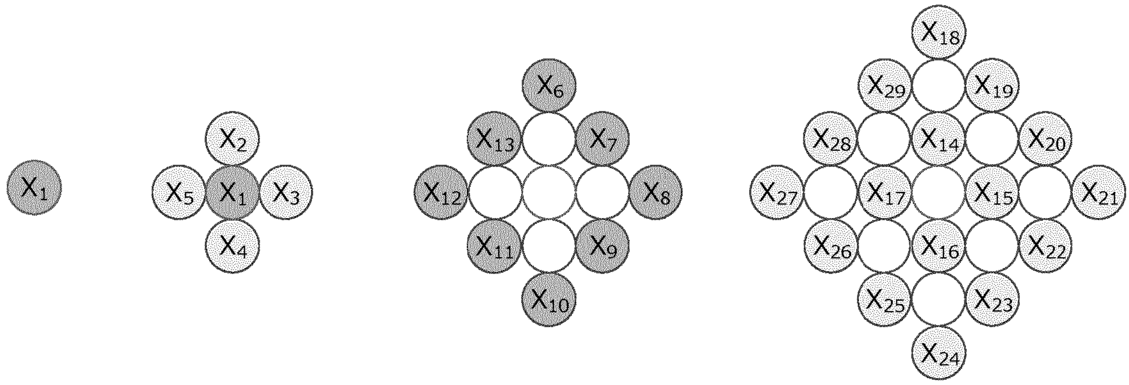


FIG. 13

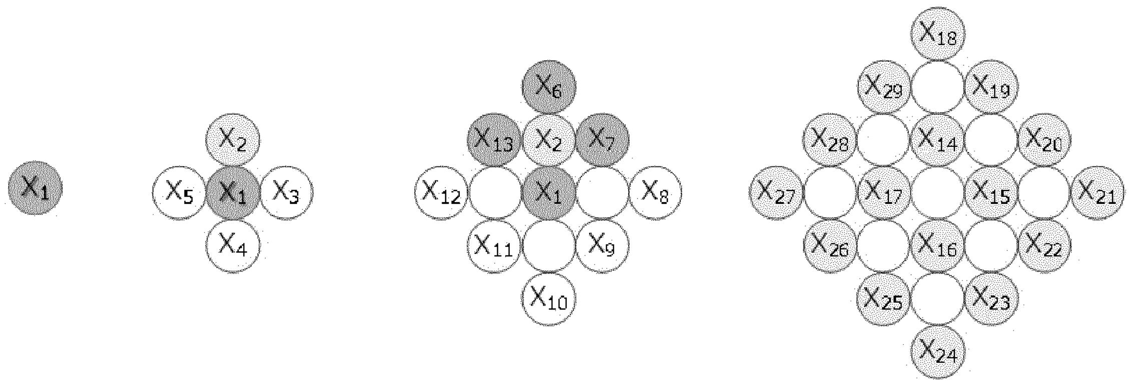


FIG. 14

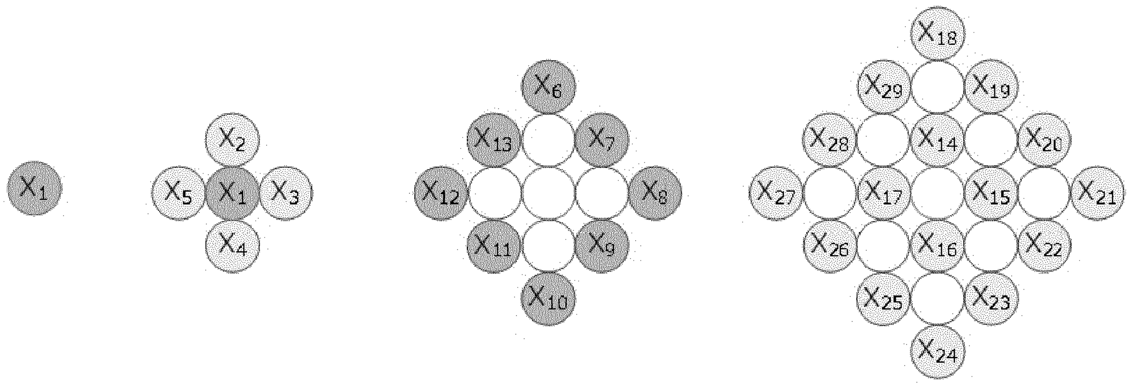

FIG. 15A

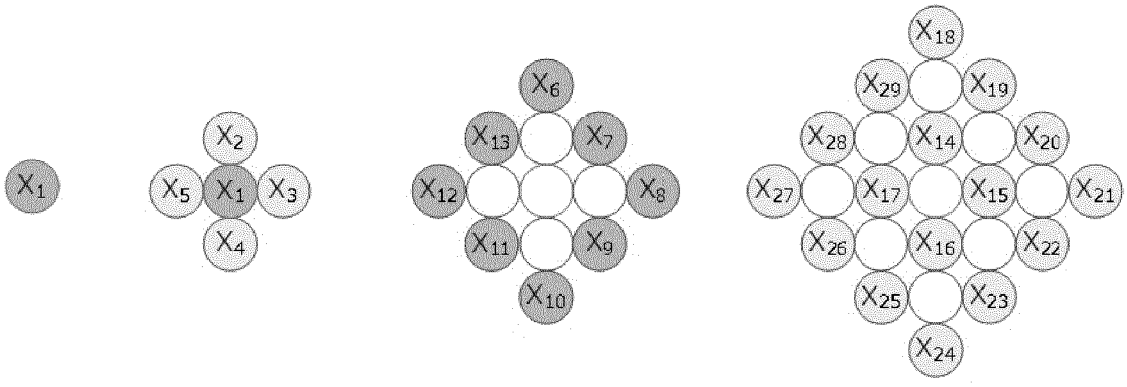


FIG. 15B

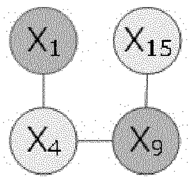


FIG. 16

| | $X_1$ | $X_2$ | $X_3$ |
|---|---|---|---|
| $X_1$ | | 2 | 0 |
| $X_2$ | 2 | | 4 |
| $X_3$ | 0 | 4 | |

FIG. 17

Optimizing device 100

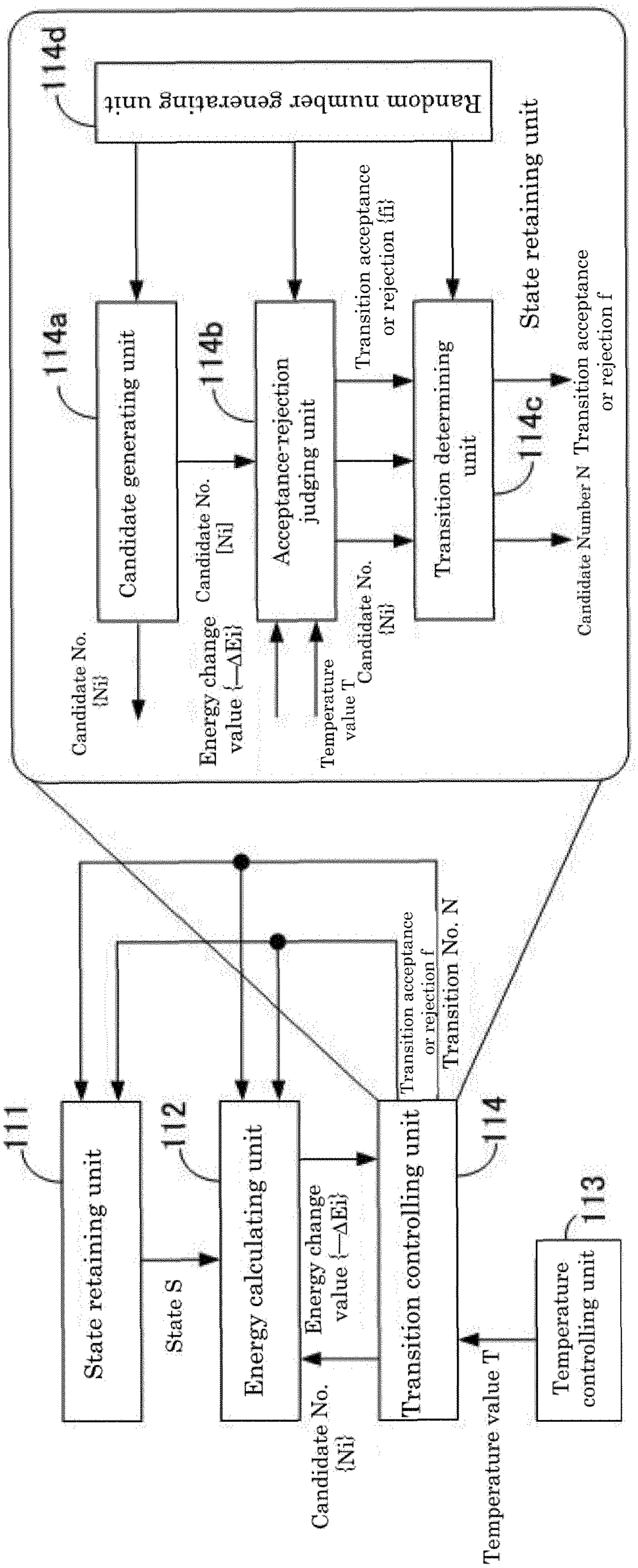

FIG. 18

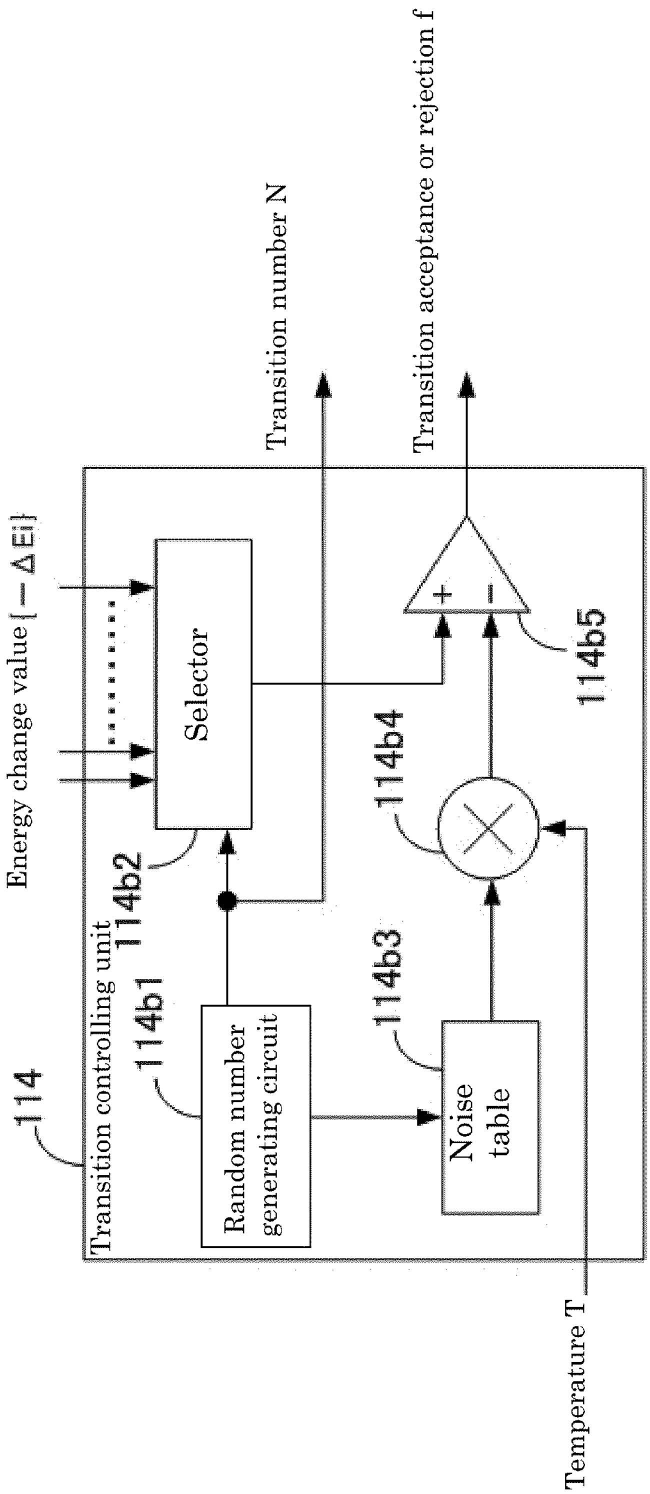
114
Transition controlling unit
114b1
Random number generating circuit
114b2
Selector
Energy change value {−ΔEi}
Transition number N
114b3
Noise table
114b4
114b5
+
-
Transition acceptance or rejection f
Temperature T

FIG. 19

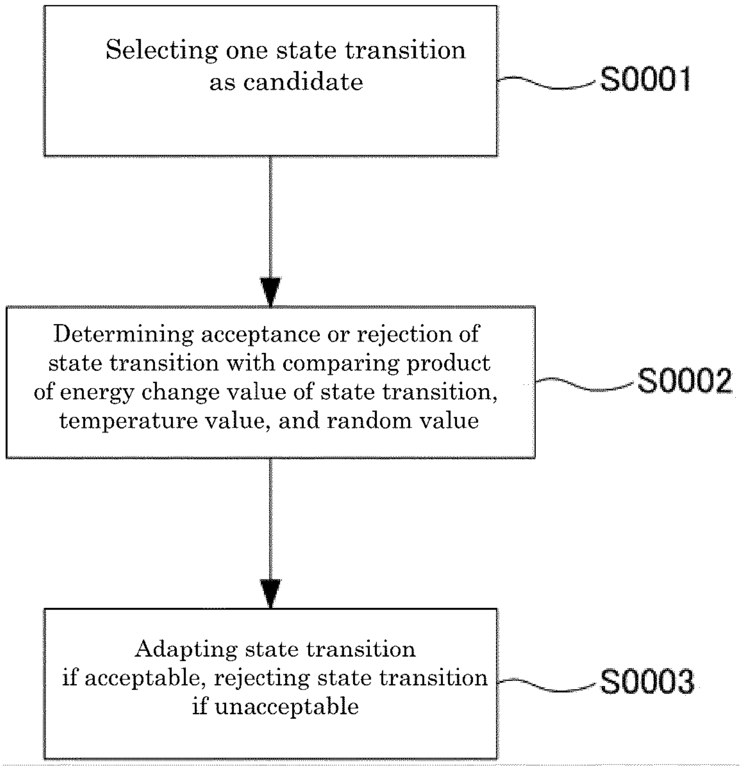


FIG. 20A

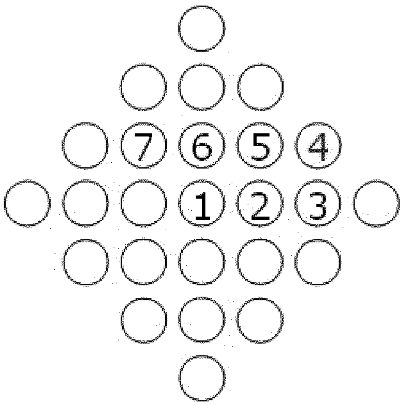

FIG. 20B

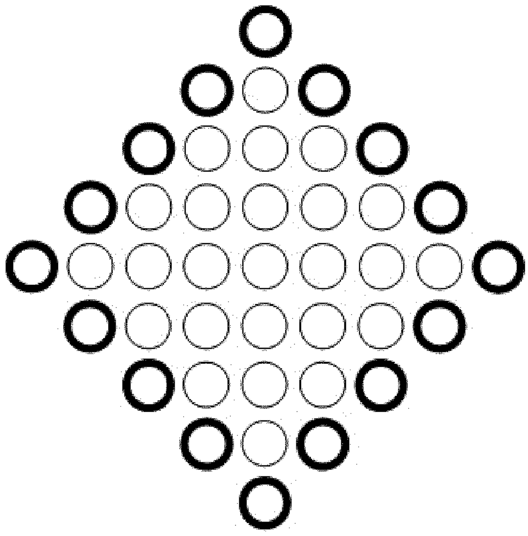

FIG. 21

Device for searching compound 10B

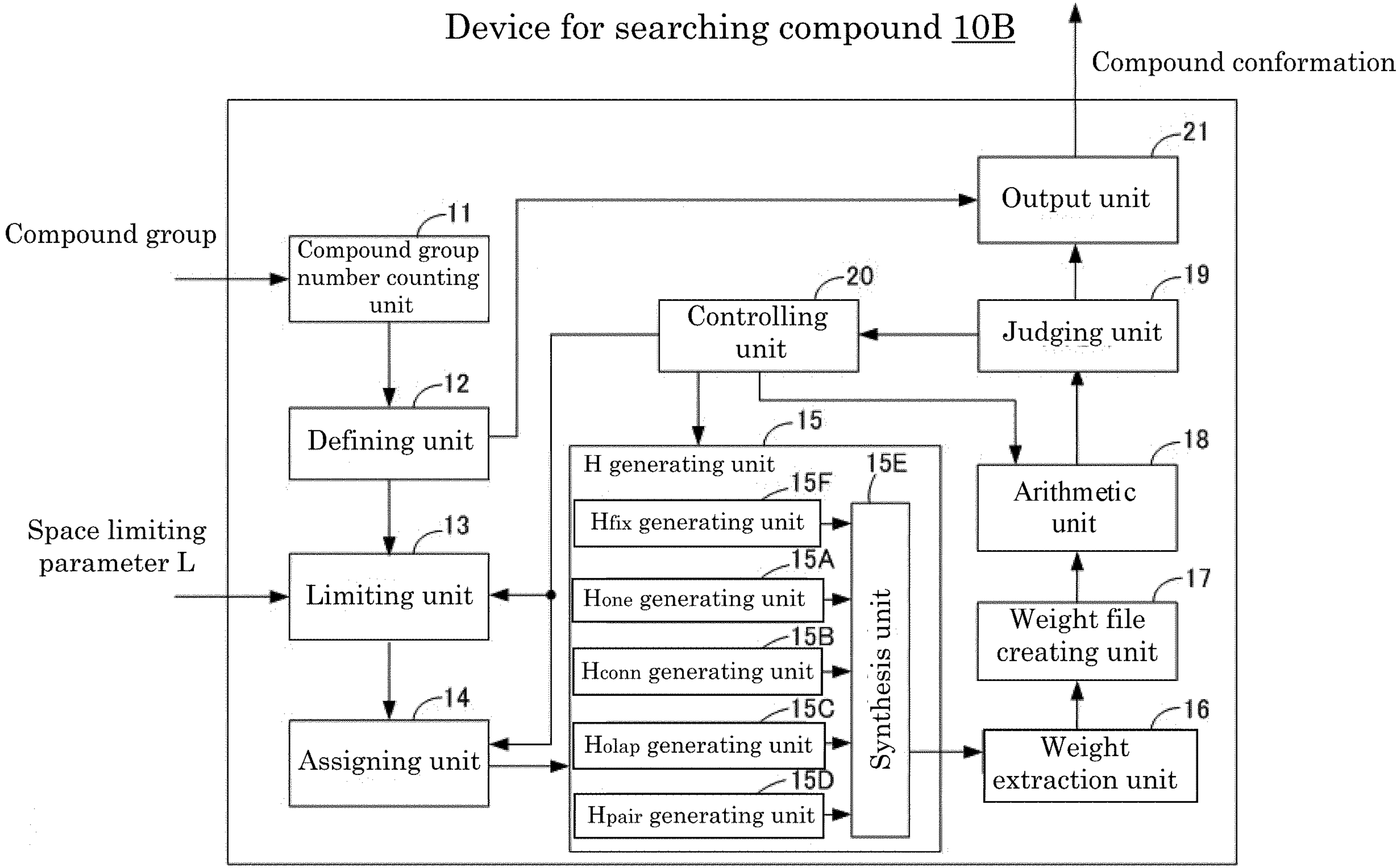

FIG. 22

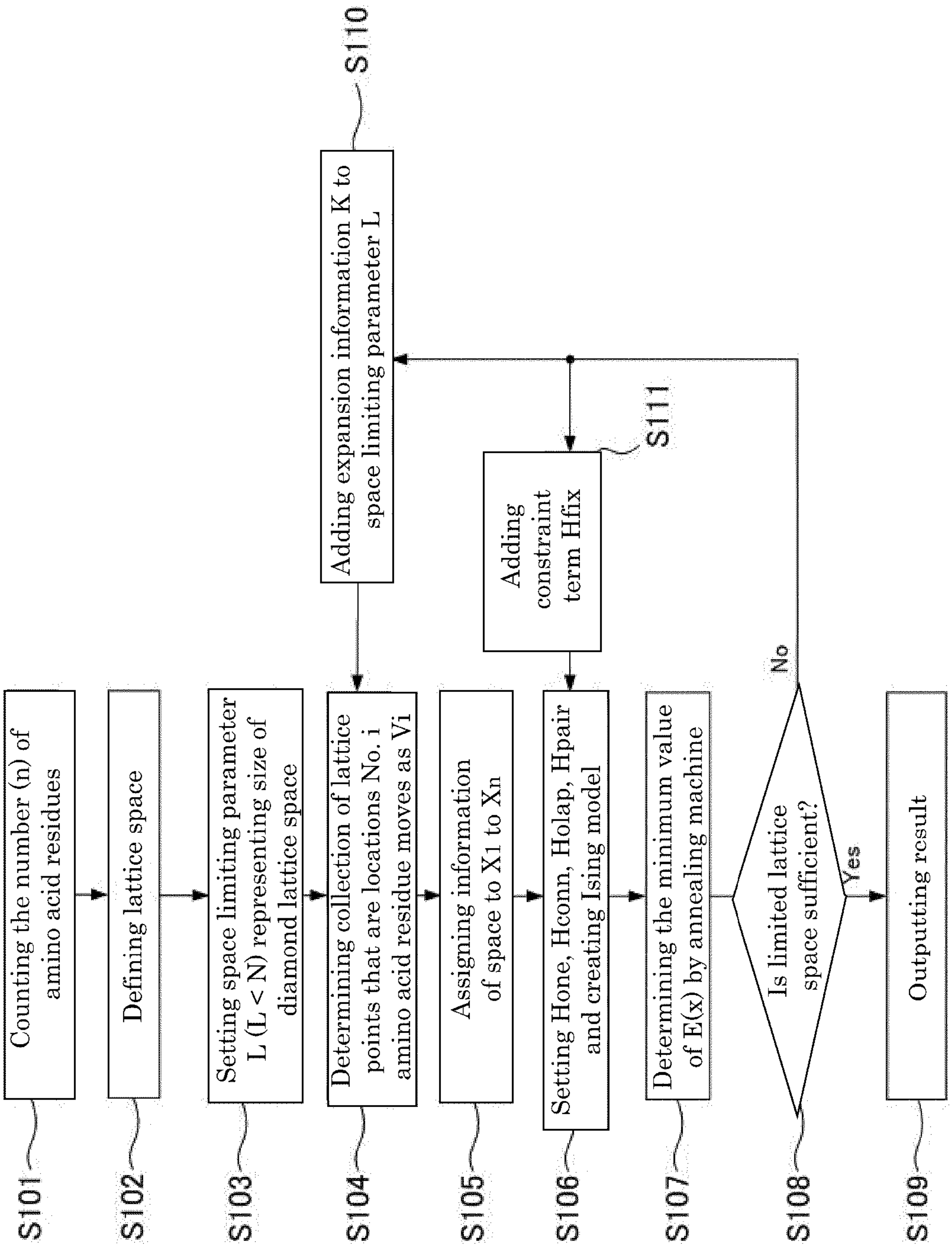
S101 Counting the number (n) of amino acid residues
S102 Defining lattice space
S103 Setting space limiting parameter L (L < N) representing size of diamond lattice space
S104 Determining collection of lattice points that are locations No. i amino acid residue moves as Vi
S105 Assigning information of space to X1 to Xn
S106 Setting Hone, Hconn, Holap, Hpair and creating Ising model
S107 Determining the minimum value of E(x) by annealing machine
S108 Is limited lattice space sufficient?
Yes
No
S109 Outputting result
S110 Adding expansion information K to space limiting parameter L
S111 Adding constraint term Hfix

FIG. 23A

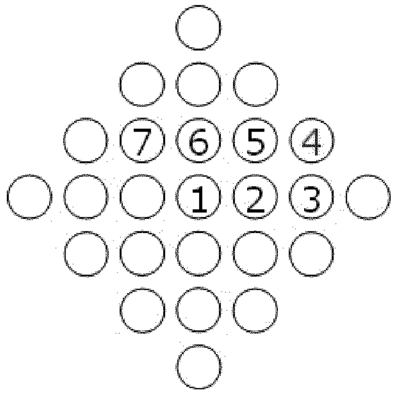


FIG. 23B

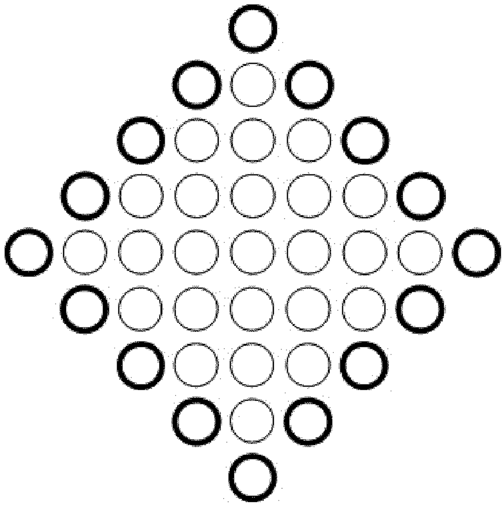

FIG. 23C

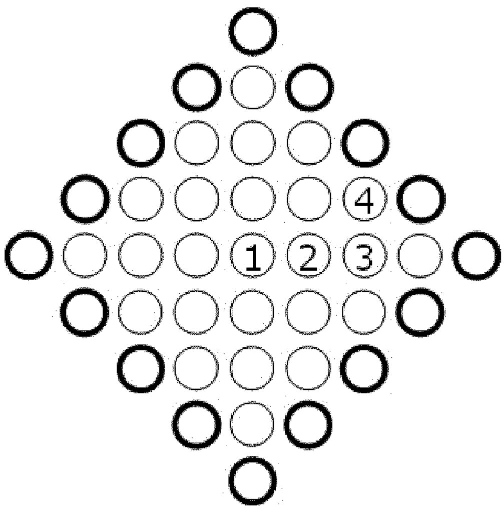

FIG.24A

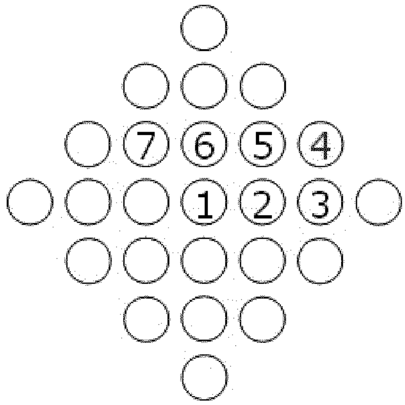


FIG. 24B

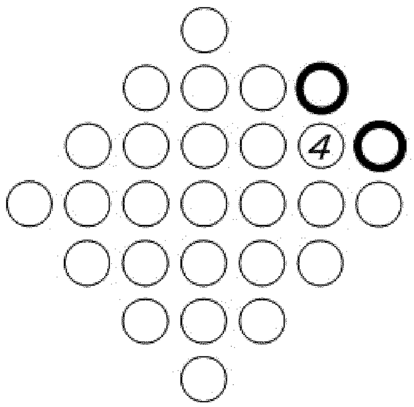


FIG. 24C

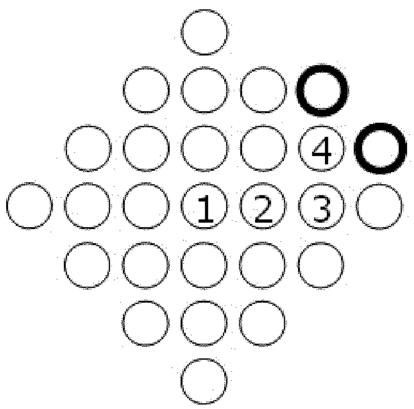

FIG. 25A

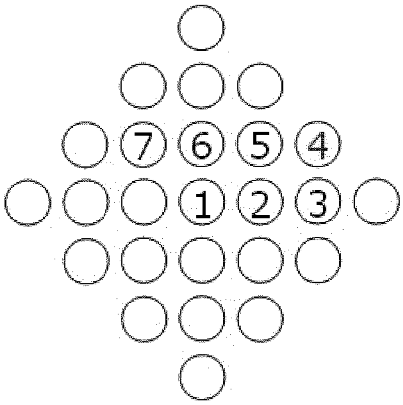


FIG. 25B

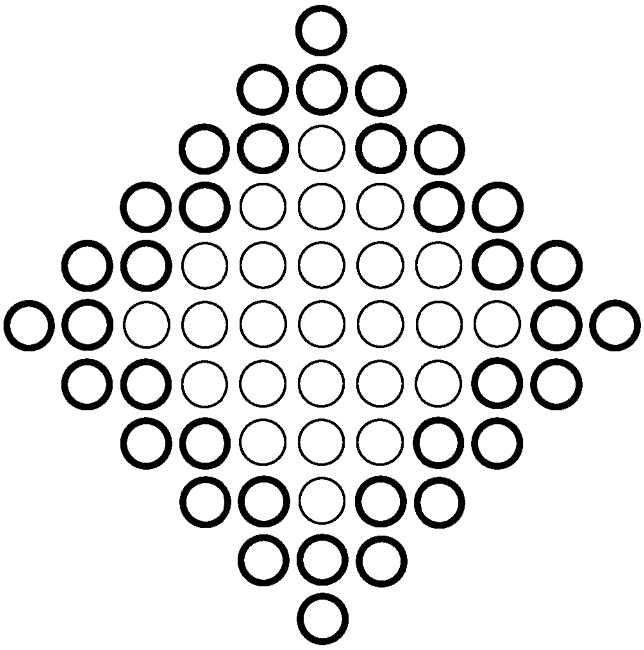

FIG. 26A

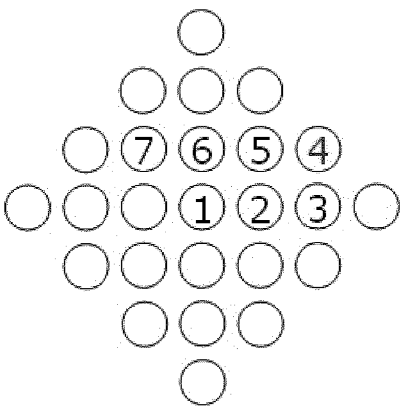

FIG. 26B

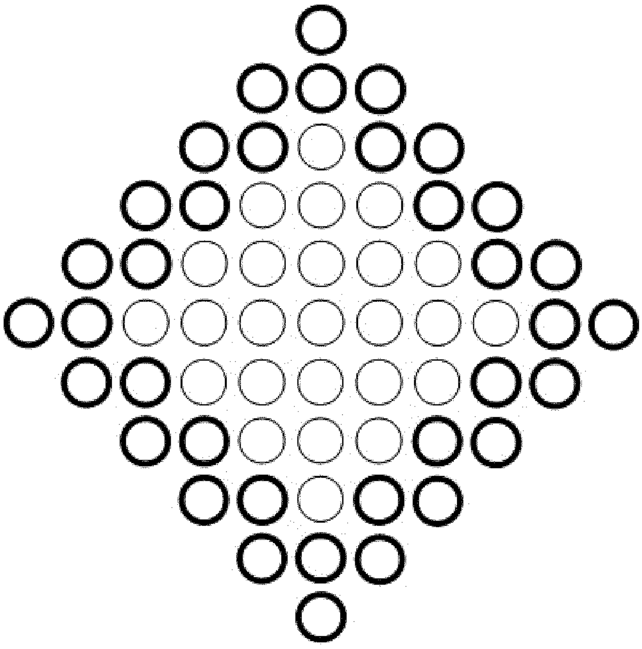

FIG. 26C

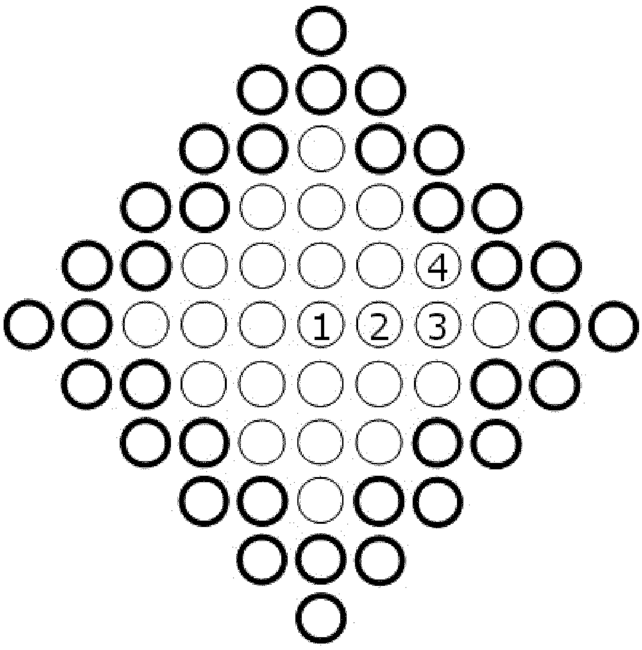

FIG. 27A

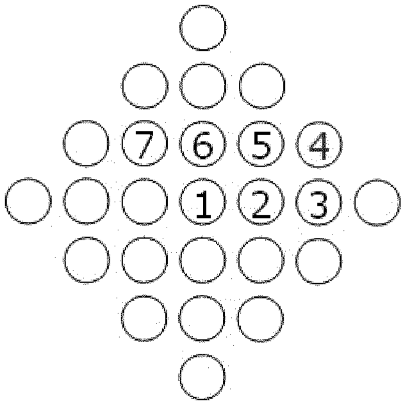


FIG. 27B

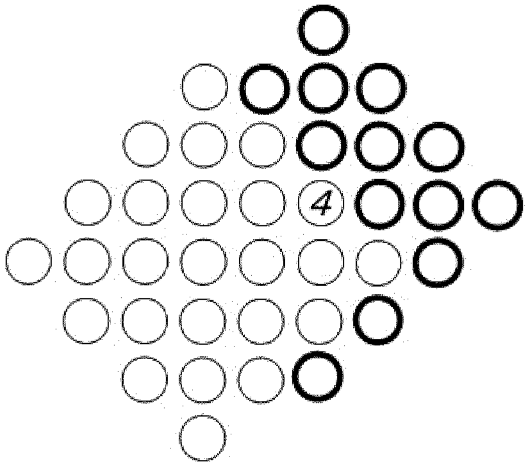


FIG. 27C

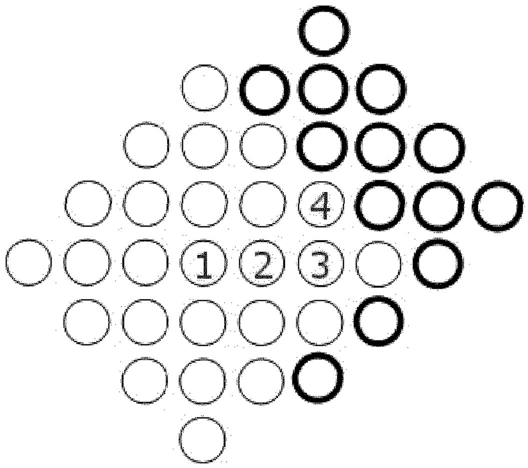

FIG. 28

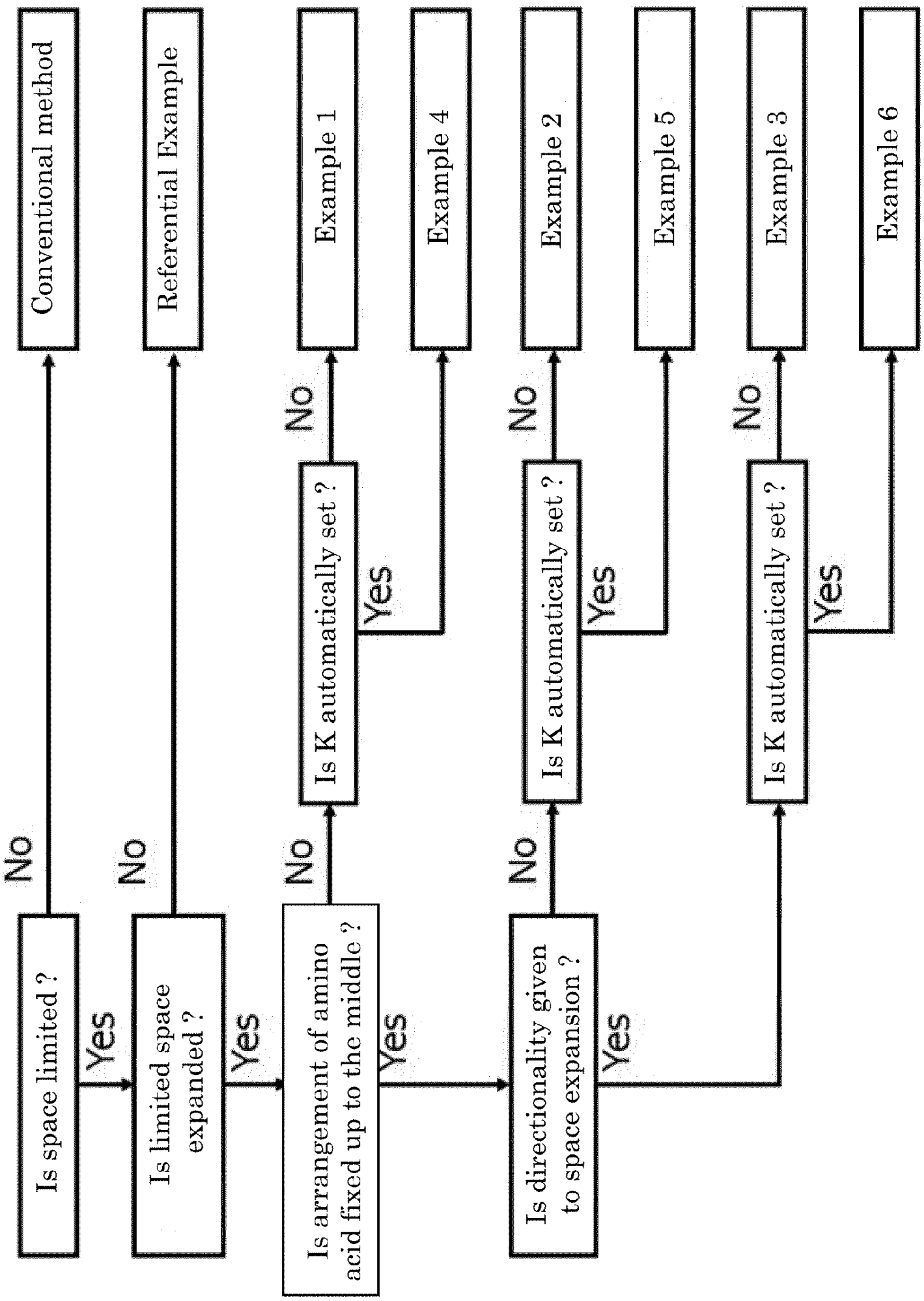

Is space limited ?
Yes
No
Conventional method
Is limited space expanded ?
Yes
No
Referential Example
Is arrangement of amino acid fixed up to the middle ?
Yes
No
Is K automatically set ?
No
Example 1
Yes
Example 4
Is directionality given to space expansion ?
Yes
No
Is K automatically set ?
No
Example 2
Yes
Example 5
Is K automatically set ?
No
Example 3
Yes
Example 6

FIG. 29

Device for searching compound 10C

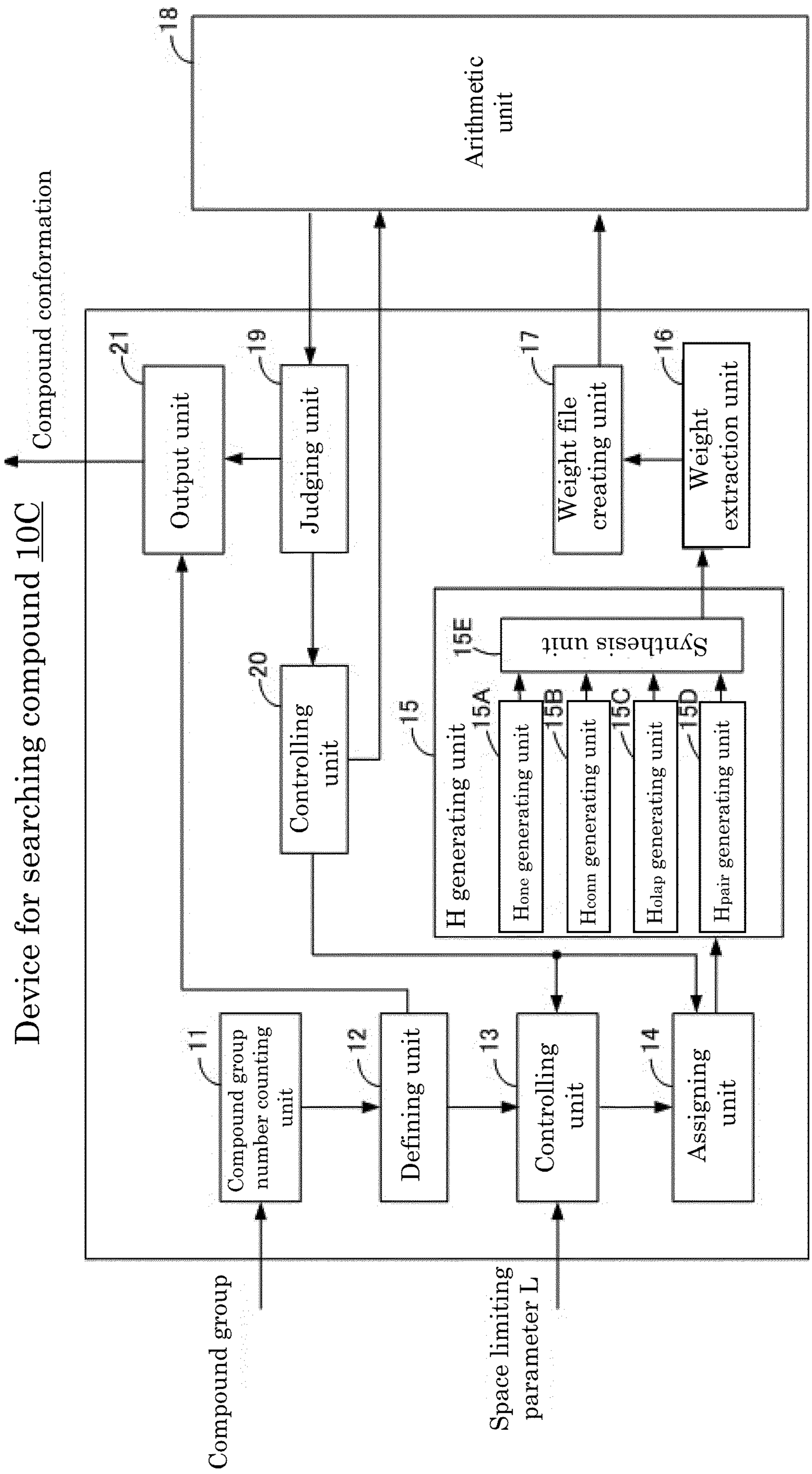

FIG. 30

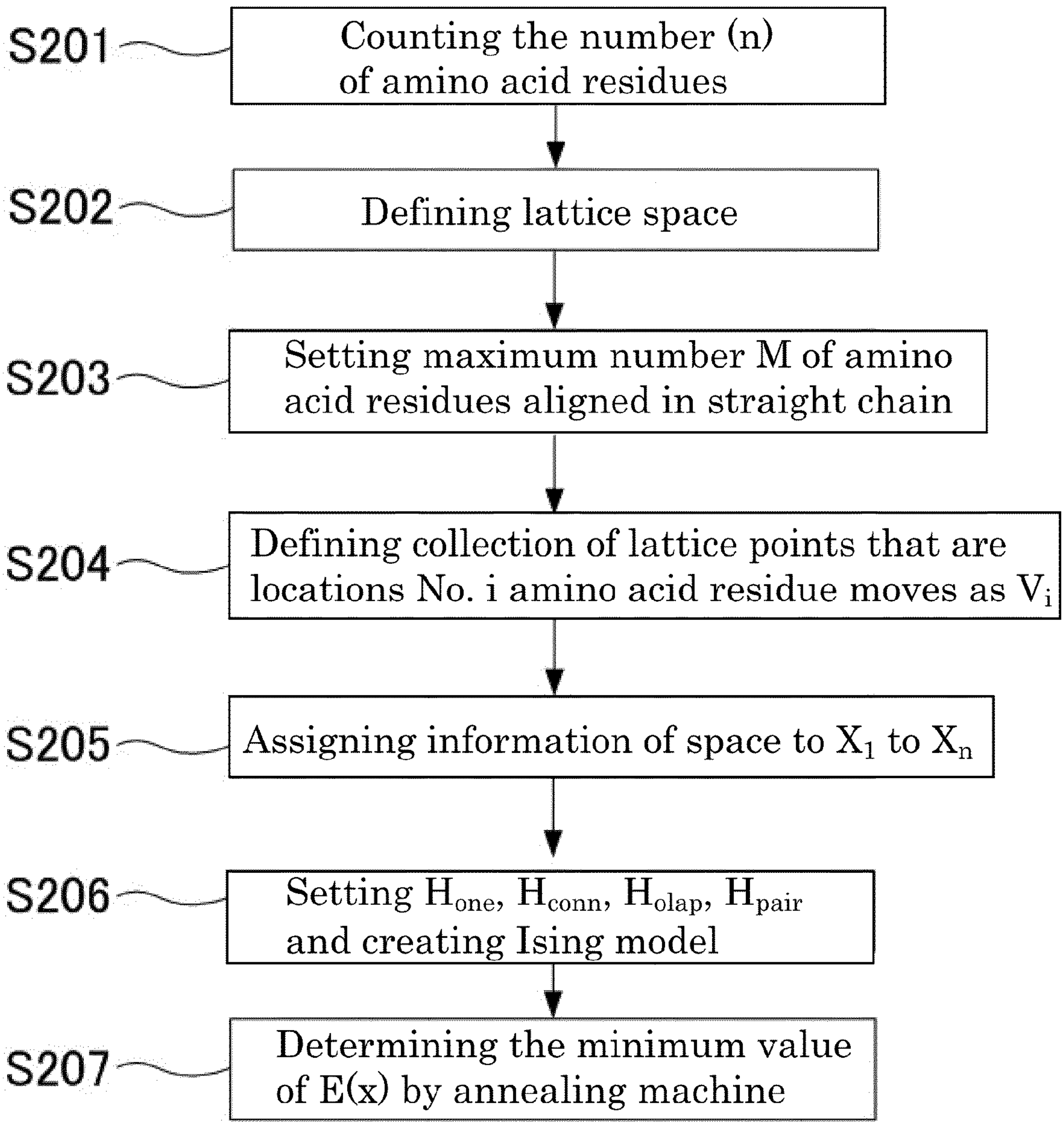
S201
Counting the number (n) of amino acid residues
S202
Defining lattice space
S203
Setting maximum number M of amino acid residues aligned in straight chain
S204
Defining collection of lattice points that are locations No. i amino acid residue moves as $V_i$
S205
Assigning information of space to $X_1$ to $X_n$
S206
Setting $H_{one}$, $H_{conn}$, $H_{olap}$, $H_{pair}$ and creating Ising model
S207
Determining the minimum value of E(x) by annealing machine

FIG. 31

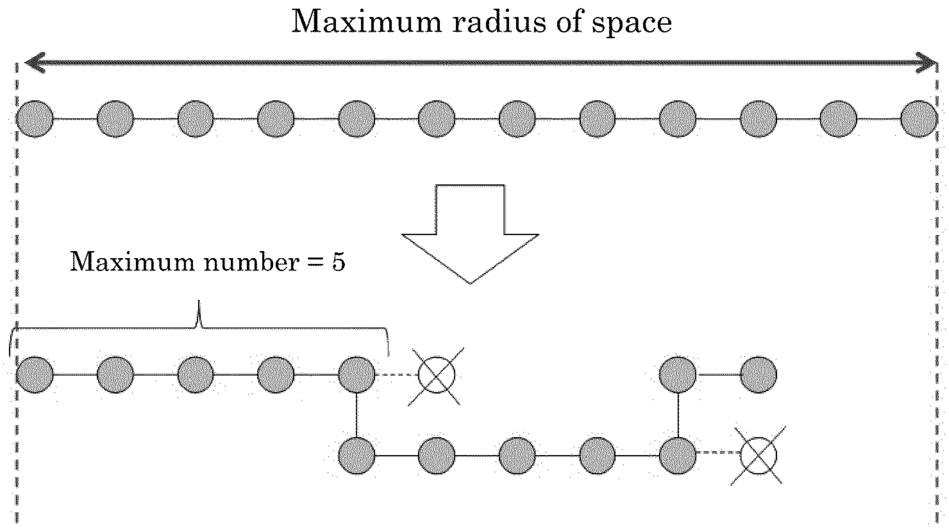
Maximum radius of space
Maximum number = 5

FIG. 32

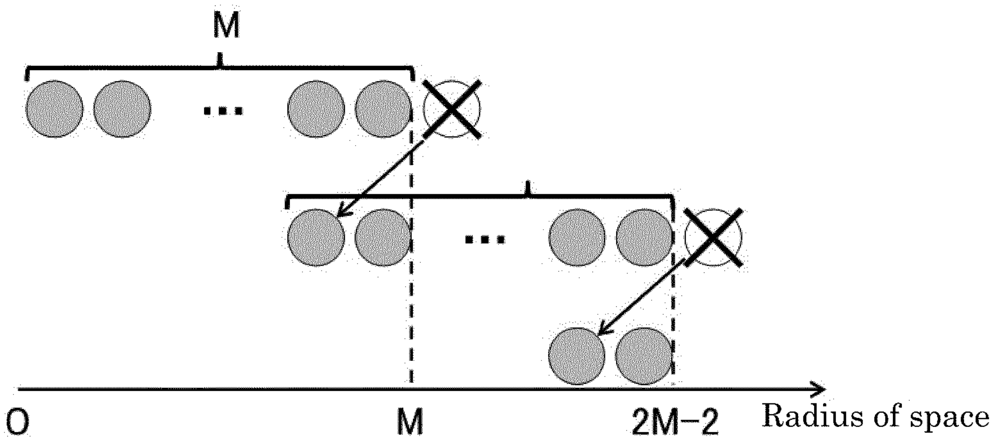
M
...
...
O
M
2M−2
Radius of space

FIG. 33

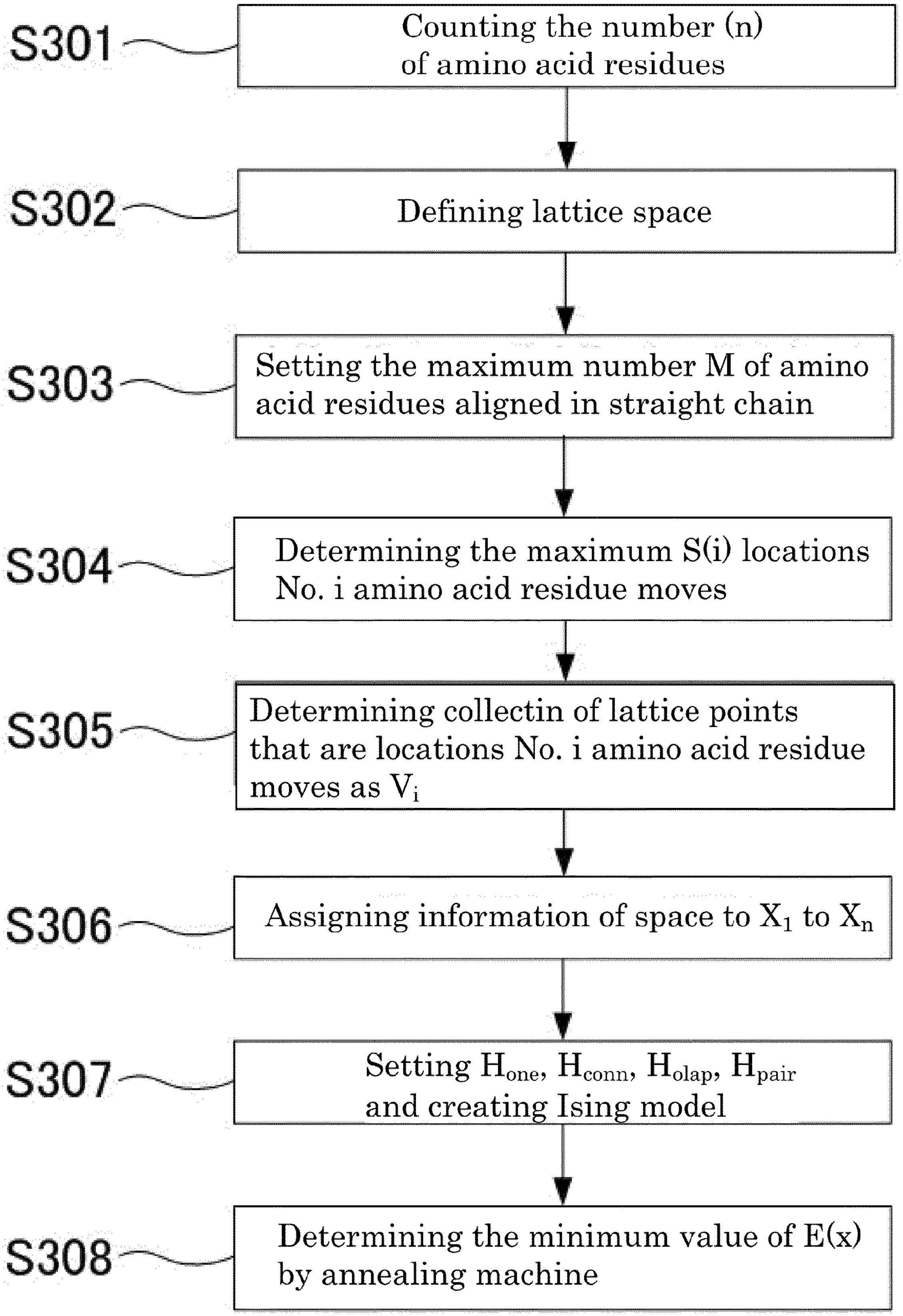
S301
Counting the number (n) of amino acid residues
S302
Defining lattice space
S303
Setting the maximum number M of amino acid residues aligned in straight chain
S304
Determining the maximum S(i) locations No. i amino acid residue moves
S305
Determining collectin of lattice points that are locations No. i amino acid residue moves as $V_i$
S306
Assigning information of space to $X_1$ to $X_n$
S307
Setting $H_{one}$, $H_{conn}$, $H_{olap}$, $H_{pair}$ and creating Ising model
S308
Determining the minimum value of E(x) by annealing machine

FIG. 34

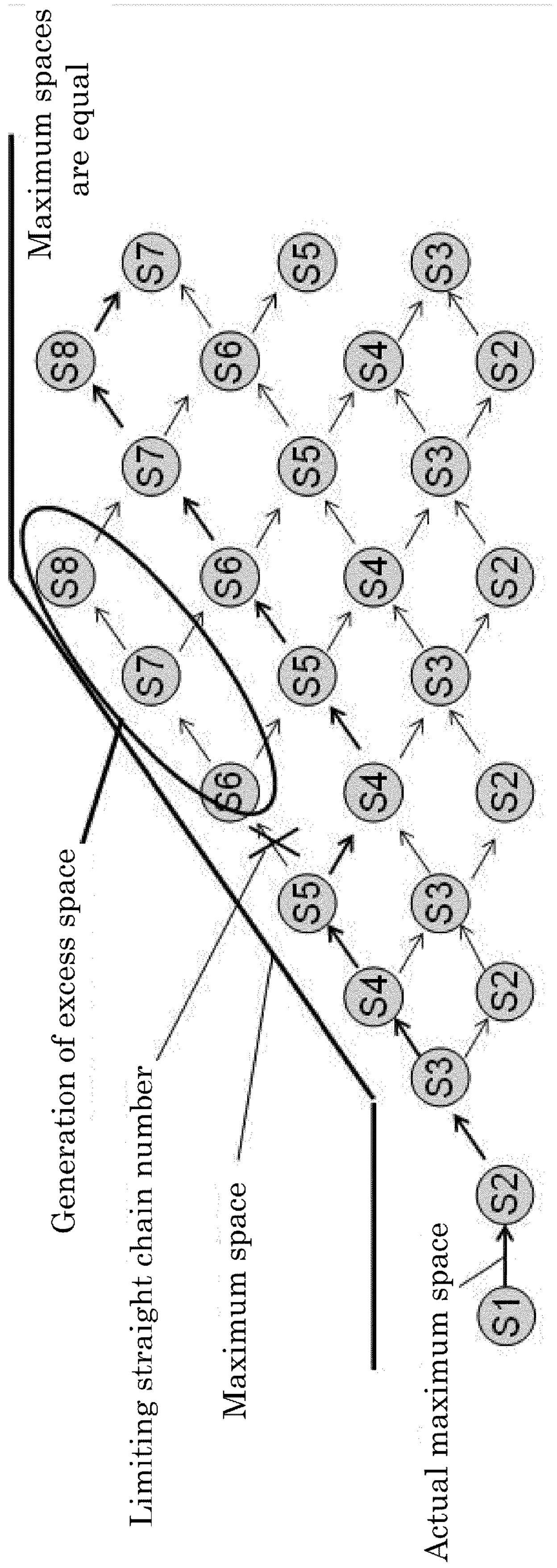

Maximum spaces are equal
Generation of excess space
Limiting straight chain number
Maximum space
Actual maximum space
S1
S2
S3
S4
S5
S6
S7
S8

FIG. 35

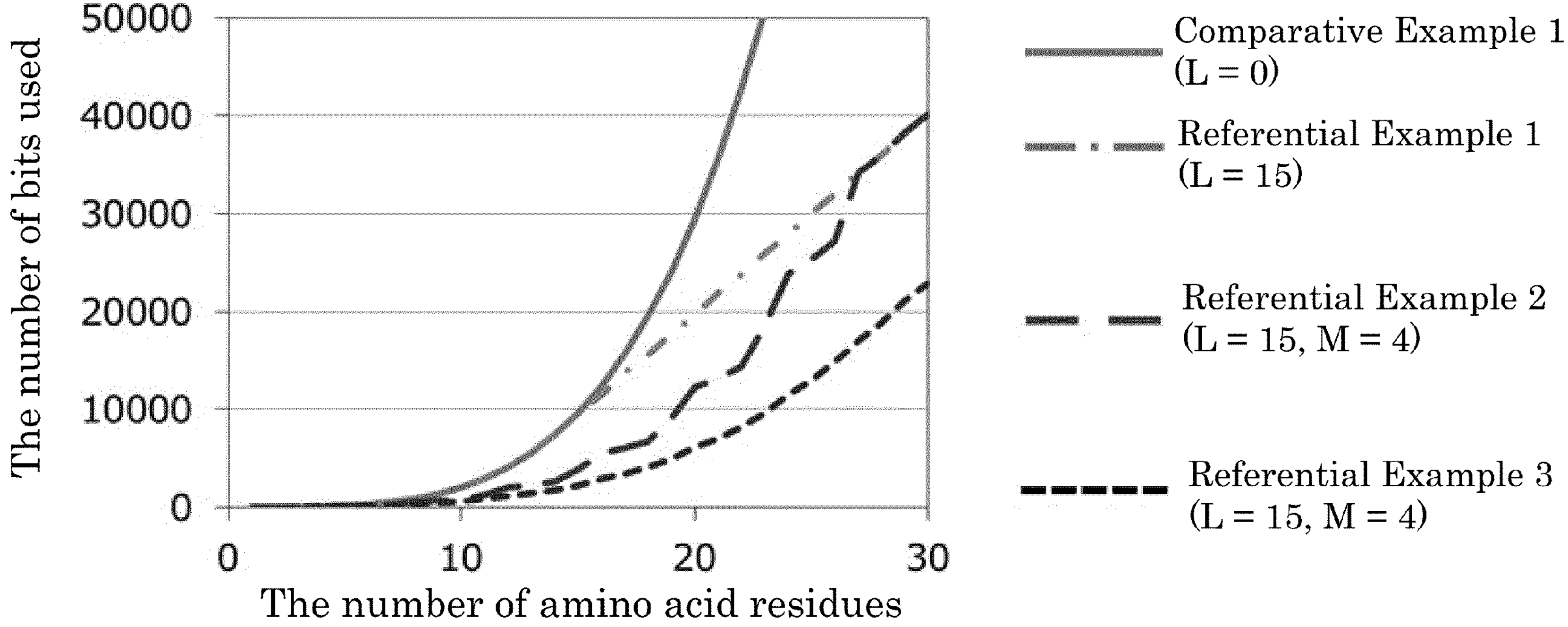
50000
40000
30000
20000
10000
0
The number of bits used
0
10
20
30
The number of amino acid residues
Comparative Example 1 (L = 0)
Referential Example 1 (L = 15)
Referential Example 2 (L = 15, M = 4)
Referential Example 3 (L = 15, M = 4)

FIG. 36

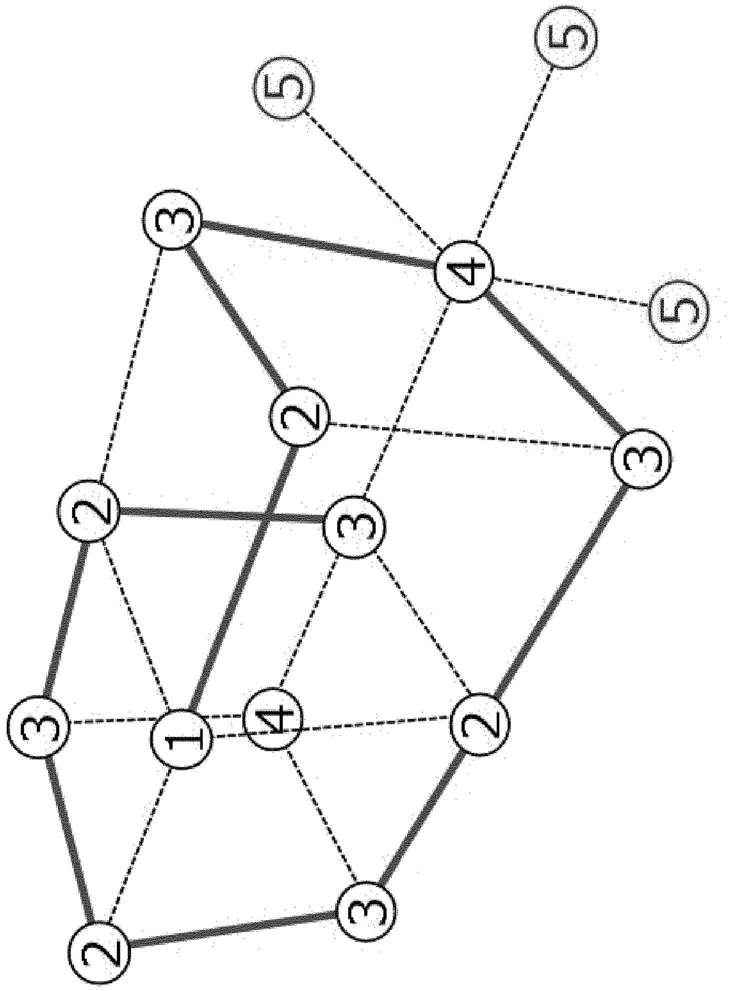

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **R. BABBUSH.** Construction of Energy Functions for Lattice Heteropolymer Models: A Case Study in Constraint Satisfaction Programming and Adiabatic Quantum Optimization. *arXiv:quant-ph/1211.3422v2, https://arxiv.org/abs/1211.3422*